# EUROPÄISCHE PATENTSCHRIFT

(11) **EP 4 037 560 B1**
(45) Veröffentlichungstag und Bekanntmachung des Hinweises auf die Patenterteilung: **26.03.2025**
(21) Anmeldenummer: 20771873.5
(22) Anmeldetag: 17.09.2020
(51) Int. Cl.: A61B 5/05, A61B 5/145, A61B 5/1455, A61B 5/00

(54) **NICHT-INVASIVE BESTIMMUNG VON GLUKOSE**
NON-INVASIVE TESTING OF GLUCOSE
DÉTERMINATION NON INVASIVE DES GLUCOSE

(30) Priorität: 30.09.2019 EP 19200618
(43) Veröffentlichungstag der Anmeldung: 10.08.2022
(73) Patentinhaber: Gluco Tera Tech AG, 6312 Steinhausen (CH)
(72) Erfinder: EBERT, Dieter, 8274 Gottlieben (CH); KLEIN, Rolf-Dieter, 80809 München (DE)
(74) Vertreter: Weickmann & Weickmann PartmbB
(86) Internationale Anmeldenummer: PCT/EP2020/076023
(87) Internationale Veröffentlichungsnummer: WO 2021/063696

(56) Entgegenhaltungen:
- JP-A- 2016 188 778
- US-A- 5 615 672
- US-A1- 2014 163 362
- US-A1- 2014 172 374
- US-A1- 2016 080 665

## Beschreibung

Die vorliegende Erfindung betrifft die nicht-invasive Bestimmung von Glucose in Blut durch Erfassung und Auswertung von Strahlung aus dem Terahertz-Bereich und dem IR-Bereich, insbesondere die nicht-invasive Bestimmung von Glucose in Kapillarblut.

Im Jahr 2016 litten etwa 415 Millionen Menschen an Diabetes. Für das Jahr 2040 ist ein Anstieg auf über 640 Millionen Menschen zu erwarten. Für Diabetes-Patienten ist eine genaue Überwachung der Konzentration von Glucose im Blut erforderlich, um eine entsprechende Medikation zu ermöglichen. Es besteht daher ein hoher Bedarf an zuverlässigen und für den Patienten einfach anwendbaren Methoden zur Bestimmung von Glucose in Blut.

Derzeit basiert die Bestimmung von Glucose in Blut überwiegend auf invasiven Methoden. Hierbei wird dem betreffenden Patienten entweder eine Blutprobe entnommen und anschließend einem *in vitro-*Test unterzogen oder es wird ein Sensor implantiert, der zur Bestimmung von Glucose *in vivo* dient. Ein Nachteil solcher invasiven Methoden ist, dass sie für den Patienten schmerzhaft und unbequem sind.

Zur Vermeidung dieses Nachteils wurden bereits zahlreiche Ansätze zur nicht-invasiven Bestimmung der Konzentration von Glucose in Blut entwickelt. Keiner dieser Ansätze hat jedoch bisher kommerzielle Bedeutung erlangt.

WO 2014/0206549 beschreibt eine Vorrichtung zum Messen von Rohdaten zur nicht-invasiven Bestimmung eines Blutparameters, beispielsweise der Konzentration von Glucose, wobei Infrarot (IR)-Strahlung aus einer externen Strahlungsquelle flächig an mehreren Messstellen in die zu untersuchende Körperoberfläche des Patienten eingekoppelt wird und die in der Körperoberfläche erzeugte IR-Strahlung an mehreren Messstellen von einer Sensoreinrichtung erfasst wird. Nachteilig an diesem Verfahren ist jedoch, dass es einen hohen apparativen Aufwand erfordert.

WO 2018/122 betrifft eine Verrichtung und ein Verfahren zur nicht-invasiven quantitativen Bestimmung eines Analyten im Blut, insbesondere zur nicht-invasiven quantitativen Bestimmung von Glucose im Kapillarblut. Hierzu wird ein ausgewählter Körperbereich mit IR-Strahlung in der Region von vorzugsweise 8-12 µm bestrahlt. Anschließend erfolgt eine selektive Auswertung der reflektierten IR-Strahlung aus oberflächennahen Blutgefäßen des bestrahlten Körperbereichs.

WO 2019/034722 betrifft ebenfalls eine Vorrichtung und ein Verfahren zur nicht-invasiven quantitativen Bestimmung eines Analyten im Blut, insbesondere zur nicht-invasiven quantitativen Bestimmung von Glucose im Kapillarblut. Dabei erfolgt eine Auswertung der vom Körper emittierten IR-Strahlung vorzugsweise in einem Wellenlängenbereich von 8-12 µm ohne Verwendung externer Strahlungsquellen.

Die Absorptionsmaxima von Glucose und anderen klinisch relevanten Analyten im Terahertz-Bereich und deren spektrometrische Bestimmung *in vitro,* werden beispielsweise von Laman et al., Biophys. J. 94 (2008), 1010-1020, Upadhya et al., J. Biol. Phys. 29 (2003), 117-121, oder Song et al., Scientic Report 8 (2018), Article Number 8964, beschrieben.

Eine weitere Vorrichtung zur Bestimmung Glukose ist aus der US 2016/080665 bekannt.

Nachteilig an bekannten Verfahren zur nicht-invasiven Bestimmung von Glucose ist jedoch deren Fehleranfälligkeit.

Durch die vorliegende Erfindung werden ein Verfahren zur nicht-invasiven Bestimmung von Glucose in einer Körperflüssigkeit und die Verwendung einer Vorrichtung in diesem Verfahren bereitgestellt, mit denen die Nachteile des Standes der Technik zumindest teilweise vermieden werden können.

Die Erfindung beruht auf der Erkenntnis, dass eine einfache, schnelle und ausreichend genaue quantitative Bestimmung von Glucose im Blut eines Testsubjekts durch nicht-invasive Methoden möglich ist. Die vorliegende Erfindung basiert auf der Erfassung von Strahlung aus dem zu untersuchenden Körperbereich eines Testsubjekts in zwei unterschiedlichen Wellenlängenregionen und kombinierte Auswertung der erhaltenen Messsignale. Hierzu erfolgen eine Bestrahlung eines vorbestimmten Körperbereichs des Testsubjekts, z.B. einer Fingerkuppe, mit Terahertz-Strahlung und eine Erfassung der reflektierten Terahertz-Strahlung in einem Terahertz-Wellenlängenbereich, in dem sich die Intensität der reflektierten Terahertz-Strahlung abhängig von der Glucose-Konzentration ändert. Zusätzlich wird aus einem vorbestimmten Körperbereich des Testsubjekts, z.B. einer Fingerkuppe, stammende körpereigene IR-Strahlung bei mindestens zwei unterschiedlichen Wellenlängen oder Wellenlängenbereichen in der Wellenlängenregion von etwa 8 µm bis etwa 12 µm separat erfasst. Die Erfassung erfolgt bei einer ersten Wellenlänge oder einem ersten Wellenlängenbereich, wo die Intensität der reflektierten IR-Strahlung von der Glucose-Konzentration m Wesentlichen unabhängig ist, und unabhängig davon bei einer zweiten Wellenlänge oder einem zweiten Wellenlängenbereich, wo sich die Intensität der reflektierten IR-Strahlung abhängig von der Glucose-Konzentration ändert. Durch differenzielle Auswertung der Messsignale von beiden IR-Wellenlängen bzw. IR-Wellenlängenbereichen in Kombination mit einer Auswertung der Messsignale im Terahertz-Bereich kann die Glucose-Konzentration bestimmt werden.

Überraschenderweise wurde festgestellt, dass eine nicht-invasive Bestimmung von Glucose im Blut durch kombinierte Erfassung und Auswertung von Strahlung aus zwei unterschiedlichen Spektralbereichen die Genauigkeit der Analytbestimmung verbessert und die Fehleranfälligkeit verringert. Insbesondere kann auf diese Weise der Einfluss von Störsubstanzen verringert werden, da solche Störsubstanzen üblicherweise nur in einem der beiden Spektralbereiche ein mit Glucose interferierendes Absorptionsverhalten aufweisen. Dadurch gelingt es unter Verwendung geeigneter Algorithmen den Beitrag der betreffenden Störsubstanz zum Messsignal quantitativ zu ermitteln, sodass eine exakte Korrektur möglich ist.

In einer Ausführungsform der Erfindung wird durch die kombinierte Bestimmung im Terahertz- und IR-Bereich der Beitrag einer Störsubstanz im IR-Messsignal durch Auswertung des Terahertz-Signals quantitativ ermittelt, wodurch ein korrigiertes IR-Messsignal erhalten wird, aus dem der Beitrag der Störsubstanz zumindest weitgehend oder vollständig eliminiert ist.

In einer weiteren Ausführungsform der Erfindung wird durch die kombinierte Bestimmung im Terahertz- und IR-Bereich der Beitrag einer Störsubstanz im Terahertz-Messsignal durch Auswertung des IR-Signals quantitativ ermittelt, wodurch ein korrigiertes Terahertz-Messsignal erhalten wird, aus dem der Beitrag der Störsubstanz zumindest weitgehend oder vollständig eliminiert ist.

In noch einer weiteren Ausführungsform der Erfindung wird durch die kombinierte Bestimmung im Terahertz- und IR-Bereich der Beitrag einer ersten Störsubstanz im Terahertz-Messsignal durch Auswertung des IR-Signals und der Beitrag einer zweiten Störsubstanz im IR-Messsignal durch Auswertung des Terahertz-Signals quantitativ ermittelt, wodurch korrigierte Terahertz- und IR-Messsignale erhalten werden, aus denen die Beiträge der ersten und der zweiten Störsubstanz zumindest weitgehend oder vollständig eliminiert sind.

Der Begriff "Terahertz-Bereich" im Rahmen der vorliegenden Erfindung bezeichnet, sofern nicht anders angegeben, einen Frequenzbereich von etwa 60 Gigahertz (GHz) bis etwa 3 Terahertz (THz) entsprechend einem Wellenlängenbereich bzw. einer Wellenlängenregion von etwa 5 mm bis etwa 0,1 mm oder einen Unterbereich davon. Beispielsweise kann der Frequenzbereich von etwa 0,3 THz bis etwa 3 THz (entsprechend einem Wellenlängenbereich bzw. einer Wellenlängenregion von etwa 1 mm bis etwa 0,1 mm) oder von etwa 60 GHz bis etwa 2,5 THz (entsprechend einem Wellenlängenbereich bzw. einer Wellenlängenregion von etwa 5 mm bis etwa 0,12 mm) reichen.

Der Begriff "IR-Bereich" im Rahmen der vorliegenden Erfindung bezeichnet, sofern nicht anders angegeben, einen Wellenlängenbereich bzw. einer Wellenlängenregion von etwa 1 µm bis etwa 20 µm oder einen Unterbereich davon. Beispielsweise kann der Wellenlängenbereich bzw. die Wellenlängenregion von etwa 5 µm bis etwa 15 µm und insbesondere von etwa 8 µm bis etwa 12 µm reichen.

Dabei erfolgen eine Bestrahlung des zu untersuchenden Körperbereichs mit Terahertz-Strahlung und eine Erfassung der reflektierten Terahertz-Strahlung mit dafür geeigneten Sensoren. Weiterhin erfolgt eine Erfassung der vom Körper emittierten IR-Strahlung mit dafür geeigneten Sensoren. Die Verwendung von externen IR-Strahlungsquellen ist dabei üblicherweise nicht vorgesehen. Zur Auswertung der Messsignale wird günstigerweise eine Vorrichtung verwendet, die von externer elektrischer und/oder thermischer Strahlung abgeschirmt ist. Dadurch wird eine signifikante Erhöhung der Messgenauigkeit erreicht.

Im Rahmen der Auswertung erfolgt weiterhin eine Messung der Temperatur des zu untersuchenden Körperbereichs, z.B. einer Fingerkuppe, und der Temperatur der zur Erfassung von Terahertz-Strahlung und IR-Strahlung verwendeten Sensoren, wobei die Temperatur der Sensoren auf einem Wert gehalten wird, der geringer als die Temperatur des zu untersuchenden Körperbereichs ist. Günstigerweise wird die Temperatur der Sensoren auf einen vorbestimmten Wert, z.B. im Bereich von 10 °C bis 25 °C, eingestellt, z.B. durch Einbettung in einen Metallblock, dessen Temperatur durch ein Temperatureinstellungselement, z.B. ein Peltier-Element, reguliert und mit einem Temperaturmesselement genau, z.B. mit einer Präzision von maximal ± 0,1 °C oder von maximal ± 0,01 °C, bestimmt wird. Die Temperatur des zu untersuchenden Körperbereichs, z.B. einer Fingerkuppe, kann - abhängig vom Testsubjekt und den äußeren Bedingungen - einen variablen Wert annehmen, der erfindungsgemäß mit einem Temperaturmesselement genau, z.B. mit einer Präzision von maximal ± 0,1 °C oder von maximal ± 0,01 °C, bestimmt wird.

Die Auswertung des Messsignals umfasst eine Temperaturkompensation der erhaltenen Messsignale unter Berücksichtigung des Werts für die Temperatur des zu untersuchenden Körperbereichs und des Werts für die Temperatur der Sensoreinheit und insbesondere der daraus resultierenden Temperaturdifferenz. Folglich betrifft die vorliegende Erfindung die nicht-invasiven Bestimmung von Glucose durch gemeinsame Auswertung von Terahertz-Strahlung und körpereigener IR-Strahlung mit Temperaturkompensation.

Ferner kann gemäß vorliegender Erfindung eine selektive Auswertung von körpereigener IR-Strahlung in der Region von etwa 8 µm bis etwa 12 µm aus oberflächennahen Blutgefäßen des untersuchten Körperbereichs erfolgen, insbesondere eine selektive Auswertung von körpereigener IR-Strahlung aus den Kapillarblutgefäßen der Dermis. Die Erfinder haben nun festgestellt, dass körpereigene IR-Strahlung aus mehreren Komponenten zusammengesetzt ist, die separat analysiert werden können. Die aus oberflächennahen Blutgefäßen stammende Komponente der körpereigenen IR-Strahlung weist eine von der arteriellen Pulsfrequenz des Testsubjekts abhängige zeitliche Variation auf. Aufgrund dieser Variation kann eine Diskriminierung zwischen sich mit der Pulsfrequenz ändernden Signalen und von der Pulsfrequenz unabhängigen Signalen im Rahmen der Auswertung vorgenommen werden.

Überraschenderweise erlaubt die vorliegende Erfindung eine genaue und reproduzierbare Bestimmung von Glucose in Blut, z.B. mit einer Genauigkeit von ± 2,5 % gegenüber einer Referenzmessung mittels Röntgendiffraktometer.

Ein erster Aspekt der vorliegenden Erfindung betrifft somit die Verwendung einer Vorrichtung zur nicht-invasiven Bestimmung von Glucose im Blut eines Testsubjekts, wie in Anspruch 1 definiert.

Die Vorrichtung enthält eine Strahlungsquelle (b) zur Erzeugung von Terahertz-Strahlung, insbesondere von Terahertz-Strahlung in einer Wellenlängenregion von etwa 0,1 mm bis etwa 5 mm, von etwa 0,12 mm bis etwa 5 mm oder von etwa 0,1 mm bis etwa 1 mm, zur Bestrahlung des zu untersuchenden Körperbereichs. Nach Bestrahlung des in die Aufnahmeeinheit (a) eingebrachten, zu untersuchenden Körperbereichs wird Terahertz-Strahlung reflektiert. Die Vorrichtung enthält weiterhin eine Einheit (c) (ii) zur Erfassung von Terahertz-Strahlung.

Die Vorrichtung enthält nicht notwendigerweise eine externe IR-Strahlungsquelle, da sie zur Erfassung und Auswertung von körpereigener IR-Strahlung eingerichtet ist. Vorzugsweise ist keine externe IR-Strahlungsquelle vorhanden. Die körpereigene IR-Strahlung wird von dem in die Aufnahmeeinheit (a) eingebrachten, zu untersuchenden Körperbereich emittiert. Die Vorrichtung enthält eine Einheit (c) (ii) zur Erfassung von körpereigener IR-Strahlung.

Die Temperatur, insbesondere die Kerntemperatur, des in die Aufnahmeeinheit eingebrachten, zu untersuchenden Körperbereichs, wird durch ein Element zur Temperaturmessung (d) (i), z.B. einen Temperatursensor, genau bestimmt. Gegebenenfalls kann ein Element (d) (ii) für die Einstellung der Körpertemperatur des zu untersuchenden Körperbereichs in der Aufnahmeeinheit (a) vorgesehen sein, d.h. ein Heiz- und/oder Kühlelement, z.B. ein Peltier-Element.

Weiterhin enthält die Vorrichtung weiterhin Mittel (e) (i), um die Temperatur in der Einheit zur Erfassung von reflektierter Terahertz-Strahlung (c) (i) und/oder der Einheit zur Erfassung von körpereigener IR-Strahlung (c) (ii) zu bestimmen, z.B. einen Temperatursensor, und Mittel (e) (ii), um die Temperatur in der Einheit zur Erfassung von reflektierter Terahertz-Strahlung (c) (i) und/oder der Einheit zur Erfassung von körpereigener IR-Strahlung (c) (ii) auf einem im Wesentlichen gleichen und vorzugsweise konstanten Temperaturniveau zu halten. Dabei ist das Element (e) (ii) zur genauen Einstellung der Temperatur der Strahlungserfassungseinheit (c) (i) und/oder (c) (ii) auf einen Wert vorgesehen, der geringer ist als der Wert der Körpertemperatur. Günstigerweise enthält das Element (e) (ii) einen Körper aus wärmeleitfähigem Material, z.B. einen Metallblock, und Mittel zur Zuleitung und/oder Ableitung von Wärme, z.B. ein Heiz- und/oder Kühlelement, wie etwa ein Peltier-Element.

Die Vorrichtung ist zur Aufnahme eines Körperbereichs eines Testsubjekts, insbesondere zur Aufnahme eines Körperbereichs eines menschlichen Testsubjekts, z.B. einer Fingerkuppe, eines Ohrläppchens oder einer Ferse oder Teilen davon, vorgesehen, um die davon emittierte körpereigene IR-Strahlung zu bestimmen. Vorzugsweise ist der zu untersuchende Körperbereich eine Fingerkuppe.

Hierzu enthält die Vorrichtung eine Einheit (a) zur Aufnahme des zu bestrahlenden Körperbereichs, die beispielsweise ein Auflageelement für den betreffenden Körperbereich, z.B. die Fingerkuppe, umfassen kann. Die Form des Auflageelements ist dem Körperbereich angepasst, an dem die Bestimmung durchgeführt wird. So kann etwa ein im Wesentlichen planares Auflageelement vorgesehen sein.

Günstigerweise ist das Auflageelement (a) teilweise gegenüber der Strahlungserfassungseinheit (c) (i) und/oder (c) (ii) thermisch isoliert, z.B. indem es zumindest teilweise aus thermisch isolierendem Material, z.B. einem Kunststoff wie etwa einem Polyurethanschaum, ausgebildet ist. Weiterhin enthält das Auflageelement einen oder mehrere Bereiche, die transparent für Terahertz-Strahlung in der Region von vorzugsweise etwa 0,1 mm bis etwa 5 mm oder von etwa 0,1 mm bis etwa 1 mm und für IR-Strahlung in der Region von vorzugsweise etwa 8 µm bis etwa 12 µm sind, um ein ungehindertes Durchdringen der von dem zu untersuchenden Körperbereich emittierten Terahertz- und IR-Strahlung zu ermöglichen. Der transparente Bereich des Auflageelements kann z.B. eine Fläche von etwa 0,5 cm² bis etwa 1,5 cm² aufweisen und z.B. kreisförmig ausgebildet sein. Beispiele für geeignete Materialien im transparenten Bereich des Auflageelements sind Silicium, Germanium oder organische Terahertz- und IR-transparente Polymere. Das Auflageelement kann in jeder geeigneten Form ausgebildet sein, beispielsweise als Scheibe.

In Verbindung mit der Aufnahmeeinheit (a) steht ein Element (d) (i) zur Messung der Temperatur in dem zu untersuchenden Körperbereich. Dieses Element umfasst einen Temperatursensor, z.B. ein Bolometer oder Thermopile. Weiterhin kann ein Temperatureinstellungselement (d) (ii) vorgesehen sein, z.B. ein Heiz- und/oder Kühlelement, insbesondere ein Peltier-Element, um gegebenenfalls eine genaue Einstellung der Temperatur des untersuchenden Körperbereichs zu ermöglichen, z.B. mit einer Präzision von maximal ± 0,1 °C oder von maximal ± 0,01 °C. Beispielsweise kann das Temperatureinstellungselement so eingerichtet sein, dass es eine Temperatur des Körperbereichs von 25 °C oder mehr, z.B. von etwa 28-38 °C einstellen kann. Weiterhin kann ein Element zur Messung der Pigmentierung des zu bestimmenden Körperbereichs (d)(iii), z.B. ein kolorimetrischer Sensor, vorhanden sein.

Vorzugsweise umfasst das Auflageelement Mittel, z.B. Sensoren, zur Erfassung und/oder Kontrolle der Auflageposition und/oder des Auflagedrucks für den zu untersuchenden Körperbereich. Dabei können die Auflageposition und/oder der Auflagedruck individuell für jeden Körperbereich erfasst und gegebenenfalls adaptiert werden. Die individuelle Adaption kann z.B. einen ein- oder mehrmaligen Abgleich des von der nicht-invasiven Vorrichtung stammenden Messsignals mit einem Referenzsignal umfassen, das konventionell, z.B. über eine invasive Bestimmung unter Verwendung eines konventionellen Teststreifens oder eines im Körper inserierten Sensors erhalten worden ist. Dieser Abgleich kann im Rahmen der Erstbenutzung der Vorrichtung erfolgen und - sofern erforderlich - in zeitlichen Abständen, z.B. täglich, jeden zweiten Tag, wöchentlich etc., wiederholt werden. Vorzugsweise umfasst der Abgleich eine Justierung von Auflageposition und/oder Auflagedruck für den zu untersuchenden Körperbereich, um ein stabiles reproduzierbares Messsignal zu erhalten, das dem Referenzsignal möglichst weitgehend entspricht. Hierzu kann die Vorrichtung Sensoren zur Erfassung und/oder Kontrolle der Auflageposition des zu bestrahlenden Körperbereichs, z.B. hinsichtlich der x-, y- und z-Koordinaten bezüglich des Auflageelements, und/oder zur Erfassung und/oder Kontrolle des Auflagedrucks, z.B. in einem Bereich von etwa 0,5-100 N, vorzugsweise im Bereich von etwa 10-50 N und besonders bevorzugt etwa 20 N enthalten. Ein Sensor zur Erfassung und/oder Kontrolle des Auflagedrucks kann z.B. eine Wägezelle enthalten. Ein Sensor zur Erfassung und/oder Kontrolle der Auflageposition kann eine Kamera, z.B. eine CCD-Kamera und/oder einen Pulssensor enthalten.

Die durch Adaption ermittelten Einstellungen für Auflageposition und/oder Auflagedruck werden vorzugsweise von der Vorrichtung registriert und gespeichert. Bei einer nachfolgenden Benutzung des Geräts können dann die korrekte Auflageposition bzw. der korrekte Auflagedruck durch ein Signal angezeigt werden, z.B. durch ein optisches und/oder akustisches Signal. Die Messung von Glucose wird erst gestartet, wenn die vorgegebenen korrekten Einstellungen verifiziert worden sind.

Die Vorrichtung enthält eine Strahlungsquelle (b) zur Erzeugung von Terahertz-Strahlung. Diese Strahlungsquelle kann zur Erzeugung kohärenter Terahertz-Strahlung ausgebildet sein. So kann Terahertz-Strahlung durch Frequenzvervielfachung oder Differenzfrequenzbildung von mehreren Lasersignalen, z.B. Distributed Feedback-Lasern, durch Quantenkaskaden-Laser, Molekülgaslaser, Freie-Elektronen-Laser, optisch-parametrische Oszillatoren und Rückwärtswellen-Oszillatoren erzeugt werden. Weiterhin können Photodioden eingesetzt werden, welche die Differenzfrequenz zweier Laser in Wechselstrom umwandeln, der durch eine geeignete fotoleitfähige Antenne in Form von Terahertz-Strahlung emittiert wird. In bestimmten Ausführungsformen der vorliegenden Erfindung kann Terahertz-Strahlung durch einen gepulsten Laser, z.B. mit einer Wellenlänge im Bereich von 500-2.000 mm, z.B. 800 mm, durch eine geeignete Terahertz-Antenne in Terahertz-Strahlung umgewandelt werden. Die Leistung eines solchen Lasers beträgt üblicherweise etwa 1 mW bis etwa 1 W.

In weiteren Ausführungsformen kann auch ein Terahertz-Sende/Empfangschip verwendet werden. Ein solcher Sende/Empfangschip kann aus Silicium- und/oder Germanium-basierten Materialien angefertigt sein. In bestimmten Ausführungsformen enthält der Chip einen oder mehrere Verstärker, z.B. Low Noise Amplifier (LNA), einen oder mehrere Quadraturmischer, einen oder mehrere Mehrphasenfilter, einen oder mehrere Frequenzteiler und/oder einen Oszillator mit mindestens einem Tuning-Eingang zur Erzeugung von Terahertz-Strahlung enthalten. Günstigerweise sind mehrere, z.B. 2, 3 oder 4 Tuning-Eingänge vorhanden, um die Bandbreite der Terahertz-Strahlung bei Bedarf zu variieren. Des weiteren können Sende- und/oder Empfangsantennen zur Abstrahlung und/oder zum Empfang von Terahertz-Strahlung auf dem Chip integriert sein. Die Leistung eines solchen Chips kann im Bereich von etwa 0.01 mW bis etwa 100 mW, insbesondere von etwa 0.1 mW bis etwa 1 mW liegen. Beispielsweise kann der Chip eine Leistung von etwa 1 mW aufweisen. Die Größe eines solchen Chips kann etwa im Bereich von bis zu etwa 100 mm², günstigerweise etwa 10 mm² bis etwa 50 mm², beispielsweise etwa 25 mm² betragen. Derartige Terahertz-Chips sind beispielsweise von Silicon Radar GmbH erhältlich.

In einer weiteren Ausführungsform der Erfindung ist eine Terahertz-Antenne vorhanden, z.B. eine Patch-Antenne oder eine Dipolantenne.

Die effektive Leistung der Terahertz-Strahlungsquelle, d.h. die auf den zu untersuchenden Körperbereich eingestrahlte Leistung, beträgt vorzugsweise etwa 1 mW bis etwa 100 mW, wobei die Strahlung kontinuierlich oder gepulst abgegeben werden kann.

Weiterhin enthält die Vorrichtung eine Einheit (c) zur Erfassung von Strahlung, und zwar eine Einheit zur Erfassung von Terahertz-Strahlung (c) (i) und eine Einheit zur Erfassung von IR-Strahlung (c) (ii).

Die Erfassungseinheit (c) (i) zur Erfassung von Terahertz-Strahlung enthält einen oder mehrere Sensoren bzw. Detektoren, die zur Erfassung von Terahertz-Strahlung, insbesondere von Terahertz-Strahlung im Frequenzbereich von etwa 60 Gigahertz (GHz) bis etwa 3 Terahertz (THz) entsprechend einem Wellenlängenbereich bzw. einer Wellenlängenregion von etwa 0,1 mm bis etwa 5 mm oder in einem Unterbereich davon, z.B im Bereich von etwa 0,12 mm bis etwa 5 mm, von etwa 0,1 mm bis etwa 1 mm oder in einem Unterbereich davon, vorgesehen sind.

In einer weiteren besonders bevorzugten Ausführungsform ist mindestens eine Fokussierlinse im Strahlengang zwischen der Terahertz-Strahlungsquelle und dem zu untersuchenden Körperbereich und/oder im Strahlengang zwischen dem zu untersuchenden Körperbereich und der Terahertz-Erfassungseinheit (c) (i) vorhanden, z.B. eine sphärische Linse, eine asphärische Linse oder eine Fresnel-Linse, die aus einem für Terahertz-Strahlung im Wesentlichen transparenten Material wie etwa Polypropylen oder HD-Polyethylen besteht. Die Linse dient zur Fokussierung der in den zu untersuchenden Körperbereich von der Terahertz-Strahlungsquelle eingestrahlten Terahertz-Strahlung auf eine vorbestimmte Eindringtiefe in den Körperbereich, z.B. von etwa 3-4 mm und/oder zur Fokussierung der vom zu untersuchenden Körperbereich reflektierten Terahertz-Strahlung auf die Terahertz-Erfassungseinheit.

Die vom Körper reflektierte Terahertz-Strahlung kann in einem oder mehreren Schritten verstärkt werden, bevor sie zur Erfassungseinheit (c) (i) geleitet wird. Typischerweise erfolgt die Verstärkung in einem ersten Schritt durch einen LNA. Danach kann die reflektierte Terahertz-Strahlung an einen Mischer geleitet und mit dem Sendesignal gemischt (multipliziert) werden. Das Mischergebnis kann anschließend weiter verstärkt werden. Beispielsweise kann eine Verstärkung um einen Faktor von 10³ oder höher, 10⁴ oder höher und bis zu 10⁶ oder 10⁷ erfolgen. In einer Ausführungsform kann das Signal in mehreren, z.B. in zwei Schritten verstärkt werden, wobei in jedem Schritt eine Signalverstärkung um einen Faktor von 10² bis 10³ erfolgen kann.

Die Einheit zur Erfassung von Terahertz-Strahlung ist eingerichtet, um Terahertz-Strahlung in einem Wellenlängenbereich zu erfassen, in dem sich die Intensität der reflektierten Terahertz-Strahlung abhängig von der Glucose-Konzentration ändert. Insbesondere ist die Einheit (c) (i) eingerichtet, ein breitbandiges Spektrum im Terahertz-Bereich, insbesondere umfassend einen Frequenzbereich von etwa 60 Gigahertz (GHz) bis etwa 3 Terahertz (THz) entsprechend einem Wellenlängenbereich bzw. einer Wellenlängenregion von etwa 0,1 mm bis etwa 5 mm, z.B. einen Wellenlängenbereich von etwa 0,1 mm bis etwa 0,25 mm (entsprechend einem Wellenzahlbereich von etwa 100 cm⁻¹ bis etwa 40 cm⁻¹ oder in einem Unterbereich davon zu erfassen.

Das Ergebnis der Terahertz-Messung kann separat oder kombiniert mit dem Ergebnis der IR-Messung aus einem Display angezeigt werden.

Weiterhin enthält die Vorrichtung eine Einheit (c) (ii) zur Erfassung von körpereigener IR-Strahlung aus dem untersuchten Körperbereich, die zur separaten Erfassung von IR-Strahlung bei mindestens zwei unterschiedlichen Wellenlängen oder Wellenlängenbereichen vorzugsweise in der Region von etwa 8 µm bis etwa 12 µm eingerichtet ist. In einer besonders bevorzugten Ausführungsform ist diese Einheit zur separaten Erfassung von IR-Strahlung bei mindestens zwei unterschiedlichen Wellenlängen oder Wellenlängenbereichen in der Region von etwa 8-10 µm eingerichtet. Zusätzlich kann ein dritter IR Sensor ohne Filter zur Vergleichsmessung als Referenztemperatur vorhanden sein.

Die Erfassungseinheit (c) (ii) zur Erfassung von IR-Strahlung enthält einen oder mehrere Sensoren, die zur Erfassung von IR-Strahlung vorgesehen sind.

In einer Ausführungsform enthält die Erfassungseinheit (c) (ii) mehrere Sensoren, die zur separaten Erfassung von IR-Strahlung mit mindestens zwei unterschiedlichen Wellenlängen oder Wellenlängenbereichen vorgesehen sind. In einer anderen Ausführungsform kann die Vorrichtung wiederum einen Sensor enthalten, der zur zeitabhängigen separaten Erfassung von IR-Strahlung mit mindestens zwei unterschiedlichen Wellenlängen oder Wellenlängenbereichen vorgesehen ist.

Dabei ist die Erfassungseinheit (c) (ii) zur separaten Erfassung von IR-Strahlung bei mindestens einer ersten Wellenlänge bzw. einem ersten Wellenlängenbereich und bei mindestens einer zweiten Wellenlänge bzw. mindestens einem zweiten Wellenlängenbereich eingerichtet. Die erste Wellenlänge bzw. der erste Wellenlängenbereich liegt vorzugsweise in der Region eines Absorptionsminimums für Glucose. Die zweite Wellenlänge bzw. der zweite Wellenlängenbereich liegt vorzugsweise in der Region eines Absorptionsbands von Glucose, d.h. in einer Region, in der die Glucose eine starke Absorption, vorzugsweise ein Absorptionsmaximum zeigt.

In einer Ausführungsform enthält die Vorrichtung mindestens einen ersten Sensor und mindestens einen zweiten Sensor, die jeweils zur separaten Erfassung von IR-Strahlung mit unterschiedlichen Wellenlängen bzw. Wellenlängenbereichen in der Region von etwa 8-12 µm eingerichtet sind. Ein erster Sensor wird dabei so ausgewählt, dass er zur Erfassung von IR-Strahlung mit einer ersten Wellenlänge bzw. mit einem ersten Wellenlängenbereich eingerichtet ist, wo die Intensität der körpereigenen IR-Strahlung von der Glucose-Konzentration im Wesentlichen unabhängig ist. Ein zweiter Sensor wird derart ausgewählt, dass er zur Erfassung von IR-Strahlung mit einer zweiten Wellenlänge bzw. einem zweiten Wellenlängenbereich eingerichtet ist, wo sich die Intensität der körpereigenen IR-Strahlung abhängig von der Glucose-Konzentration ändert.

Die Vorrichtung kann jeweils einen oder mehrere erste und zweite IR-Sensoren enthalten. In einer spezifischen Ausführungsform enthält die Vorrichtung einen ersten Sensor, der zur Erfassung von IR-Strahlung eingerichtet ist, deren Intensität von der Glucose-Konzentration unabhängig ist und zwei oder mehr zweite Sensoren, die zur Erfassung von IR-Strahlung eingerichtet sind, deren Intensität sich abhängig von der Glucose-Konzentration ändert, wobei die zwei oder mehr zweiten Sensoren zur Erfassung von IR-Strahlung mit jeweils unterschiedlichen Wellenlängenbereichen eingerichtet sind.

Die ersten und/oder zweiten IR-Sensoren können als Wellenlängenunspezifische Strahlungssensoren, beispielsweise als Bolometer oder Thermopiles, ausgebildet sein, die mit optischen Filterelementen ausgestattet sind, die für eine jeweils zu erfassende Wellenlänge bzw. einen zu erfassenden Wellenlängenbereich durchlässig sind, um auf diese Weise eine Wellenlängen(bereichs)-spezifische Erfassung der IR-Strahlung zu ermöglichen. Hierzu können geeignete Filterelemente, z.B. Bandpass-, Hochpass- oder Tiefpassfilterelemente oder Kombinationen mehrerer solcher Filterelemente verwendet werden. Vorzugsweise sind die optischen Filterelemente so angeordnet, dass sie direkt, d.h. ohne Abstand, auf den Sensoren aufliegen. In einer bevorzugten Ausführungsform werden als erster und/oder zweiter Sensor Bolometer oder Thermopiles mit hoher Präzision verwendet.

In einer Ausführungsform können zur Wellenlängen-spezifischen Erfassung von IR-Strahlung der erste und/oder der zweite IR-Sensor mit engen Bandpassfilterelementen ausgestattet sein, die eine Durchlässigkeitsbreite von jeweils z.B. bis zu 0,8 µm, bis zu 0,6 µm, bis zu 0,4 µm, bis zu 0,3 µm oder bis zu 0,2 µm um die erste oder zweite Messwellenlänge aufweisen.

In noch einer weiteren Ausführungsform können die Filterelemente des ersten und/oder des zweiten IR-Sensors Kombinationen von weiten Bandpassfilterelementen mit einer Durchlässigkeitsbreite von z.B. 2-12 µm, vorzugsweise 3-8 µm, Hochpassfilterelementen und/oder Tiefpassfilterelementen umfassen. So kann ein weites Bandpassfilterelement vorgesehen sein, das für IR-Strahlung in einer den gesamten Messwellenlängenbereich überdeckenden Region durchlässig ist, beispielsweise in einer Region von 8-10,5 µm oder 7-14 µm. Dieses Bandpassfilterelement kann in Kombination mit Tiefpass- und/oder Hochpassfilterelementen eingesetzt werden, um für den ersten und zweiten Sensor jeweils eine separate Erfassung von IR-Strahlung unterschiedlicher Wellenlängen oder Wellenlängenbereichen zu ermöglichen. Dabei kann einer der beiden Sensoren ein Tiefpassfilterelement enthalten, welches für IR-Strahlung bis hinab zu einer Grenzwellenlänge durchlässig ist, die zwischen der ersten Wellenlänge bzw. dem ersten Wellenlängenbereich und der zweiten Wellenlänge bzw. dem zweiten Wellenlängenbereich liegt. Alternativ oder zusätzlich kann der andere der beiden Sensoren ein Hochpassfilterelement enthalten, das für IR-Strahlung bis hinauf zu einer zweiten Grenzwellenlänge durchlässig ist, die ebenfalls zwischen der ersten Messwellenlänge bzw. dem ersten Messwellenlängenbereich und der zweiten Messwellenlänge bzw. dem zweiten Messwellenlängenbereich liegt.

Der Vorteil einer Verwendung von breiten Bandpassfiltern in Kombination mit einem Tiefpass- und/oder Hochpassfilter besteht darin, dass breitere Wellenlängenbereiche erfasst werden können. Auf diese Weise kann die Effizienz der Messung deutlich erhöht werden.

In einer konkreten Ausgestaltung dieser Ausführungsform können einer der beiden IR-Sensoren ein weites Bandpassfilter in Kombination mit einem Tiefpassfilter und der andere der ersten oder zweiten Sensoren ein breites Bandpassfilter in Kombination mit einem Hochpassfilter enthalten. In einer zweiten Ausgestaltung kann einer der ersten oder zweiten IR-Sensoren gegebenenfalls nur ein breites Bandpassfilter und der andere der ersten oder zweiten Sensoren die Kombination eines Bandpassfilters mit einem Tiefpassfilter oder alternativ die Kombination eines Bandpassfilters mit einem Hochpassfilter enthalten. Bei den beiden letztgenannten Ausgestaltungsformen überlappen die von den ersten oder zweiten Sensoren erfassten Wellenlängenbereiche, so dass bei der Auswertung dieser überlappende Bereich herausgerechnet werden muss.

In noch einer weiteren Ausführungsform können die ersten IR-Sensoren und/oder die zweiten IR-Sensoren auch als Wellenlängen(bereichs)-spezifische Sensoren, beispielsweise als Quantenkaskaden-Sensoren ausgebildet sein.

In einer anderen Ausführungsform enthält die Erfassungseinheit (c) (ii) mindestens einen IR-Sensor, der zur zeitabhängigen separaten Erfassung von IR-Strahlung mit mindestens zwei unterschiedlichen Wellenlängen bzw. Wellenlängenbereichen in der Region von etwa 8-12 µm, eingerichtet ist,
wobei bei einer ersten Wellenlänge bzw. einem ersten Wellenlängenbereich die Intensität der körpereigenen IR-Strahlung von der Glucose-Konzentration im Wesentlichen unabhängig ist, und
wobei bei einer zweiten Wellenlänge bzw. einem zweiten Wellenlängenbereich sich die Intensität der körpereigenen IR-Strahlung abhängig von der Glucose-Konzentration ändert.

Dabei kann der zur zeitabhängigen separaten Erfassung von Strahlung mit unterschiedlichen Wellenlängen oder Wellenlängenbereichen vorgesehene Sensor als Fabry-Perot Interferometer ausgebildet sein, beispielsweise als MEMS-Spektrometer für den MIR/TIR-Bereich von etwa 3-12 µm (siehe z.B. Tuohinieni et al., J. Micromech. Microeng. 22 (2012), 115004; Tuohinieni et al., J. Micromech. Microeng. 23 (2013), 075011).

Zusätzlich kann die Erfassungseinheit (c) (ii) gegebenenfalls noch mindestens einen weiteren Sensor, z.B. ein Bolometer oder Thermopile, enthalten, der zur unspezifischen Erfassung von körpereigener IR-Strahlung aus dem bestrahlten Körperbereich des Testsubjekts eingerichtet ist und zur Referenzierung, z.B. zur Referenzierung der Körpertemperaturdienen kann. Dieser weitere Sensor kann auch die Funktion des Temperaturmesselements (d) (i) wahrnehmen.

Die Größe der Sensoren in den Erfassungseinheiten (c) (i) und/oder (c) (ii) der Vorrichtung kann je nach Bedarf gewählt werden. Beispielsweise können sie eine Querschnittsfläche im Bereich von 0,5-10 mm² aufweisen.

Ein oder mehrere Sensoren können gegebenenfalls in Form eines Arrays aus mehreren einzelnen Sensorelementen wie etwa Bolometern oder Thermopiles, wie etwa mit 4-100 Einzelelementen in einer Anordnung von z.B. 2 × 2, 3 × 3, 4 × 4 oder 8 × 8 Einzelelementen vorliegen, wobei die einzelnen Sensorelemente innerhalb eines Arrays unmittelbar benachbart angeordnet oder durch Zwischenräume voneinander getrennt sein können.

Die Sensoren der Vorrichtung, insbesondere die Sensoren der Erfassungseinheiten (c) (i) und (c) (ii), befinden sich günstigerweise in einem Zustand der thermischen Äquilibrierung, d.h. sie weisen ein im Wesentlichen gleiches Temperaturniveau auf. Beispielsweise können die Sensoren in Kontakt mit einem gemeinsamen wärmeleitfähigen Träger wie etwa einem Körper, einem Block, einer Platte oder einer Folie aus Metall, z.B. Kupfer oder Messing, stehen, z.B. indem sie darin eingebettet sind. So können die Sensoren in Vertiefungen eines Körpers oder eines Blocks angeordnet sein, wobei jeweils ein Sensor in einer Vertiefung oder mehrere Sensoren in einer Vertiefung angeordnet sein können. Der Träger bzw. Block kann wiederum in Verbindung mit einem Element zur Zuleitung und/oder Ableitung von Wärme, z.B. einem Heiz- und/oder Kühlelement, wie etwa ein Peltier-Element stehen.

In einer bevorzugten Ausführungsform sind die Sensoren, insbesondere die Sensoren der Erfassungseinheiten (c) (i) und (c) (ii) in einen Metallblock eingebettet, z.B. in einen Kupferblock, der gegebenenfalls mit Gold beschichtet ist.

Eine besonders bevorzugte Ausführungsform kann 3 oder 4 oder mehr Sensoren enthalten, die z.B. gemeinsam in einer Vertiefung eines Metallblocks angeordnet sind, wobei jeder dieser Sensoren als Array von mehreren einzelnen Sensorelementen, z.B. 8 × 8 Einzelelementen, ausgebildet sein kann. Einer dieser Sensoren ist für die Erfassung von IR-Strahlung im Bereich eines Absorptionsminimums des zu bestimmenden Analyten vorgesehen, einer oder zwei dieser Sensoren sind zur Erfassung von IR-Strahlung im Bereich eines Absorptionsbandes des zu bestimmenden Analyten vorgesehen. Gegebenenfalls kann ein weiterer Sensor vorgesehen sein, der zur Referenzierung dient.

In noch einer weiteren Ausführungsform sind nur die Sensoren der Erfassungseinheit (c) (ii), d.h. die IR-Sensoren in einem Zustand der thermischen Äquilibrierung, während die Sensoren der Erfassungseinheit (c) (i), d.h. die Terahertz-Sensoren ungekühlt sind.

In Verbindung mit der Strahlungserfassungseinheit (b) steht ein Element (d) (i) zur Messung deren Temperatur und ein Element (d) (ii) zur Einstellung deren Temperatur, insbesondere der Temperatur im Bereich der Sensoren. Das Element zur Temperaturmessung (d) (i) kann z.B. als Temperatursensor ausgebildet sein. Beispielsweise kann als Element zur Temperaturmessung ein Infrarot-Kalibrator bzw. Schwarzstrahler verwendet werden. Das Element zur Temperatureinstellung (d) (ii) kann z.B. als Heiz und/oder Kühlelement, insbesondere als Peltier-Element, ausgebildet sein.

Bevorzugt ist das Element zur Temperatureinstellung, z.B. das Peltier-Element, mit seiner kühlenden Seite der Strahlungserfassungseinheit (c) zugewandt und weist auf der wärmenden, von der Strahlungserfassungseinheit (c) abgewandten Seite eine Wärmeableitung, z.B. ein oder mehrere Wärmerohre wie etwa Heatpipes oder Thermosiphons, auf. Das Element zur Temperatureinstellung ist eingerichtet, um in der Erfassungseinheit (c) und insbesondere im Bereich der Terahertz- und IR-Sensoren ein Wert für die Temperatur vorzusehen, der geringer als der Wert für die Temperatur, insbesondere für die Kerntemperatur des zu untersuchenden Körperbereichs, z.B. der Fingerkuppe, ist. Vorzugsweise wird die Temperatur in der Erfassungseinheit auf einen Wert im Bereich von 10 °C bis 25 °C eingestellt und mit einem Element zur Temperaturmessung bestimmt. Temperatureinstellung und Temperaturmessung in der Erfassungseinheit erfolgen günstigerweise mit einer Präzision von maximal ± 0,1 °C oder von maximal ± 0,01 °C. Auch die Messung der Temperatur des zu untersuchenden Körperbereichs erfolgt günstigerweise mit einer Präzision von maximal ± 0,1 °C oder von maximal ± 0,01 °C. Der Temperaturunterschied zwischen den Terahertz- und IR-Sensoren der Erfassungseinheit und des zu untersuchenden Körperbereichs kann daher mit hoher Präzision, z.B. von maximal ± 0,2 °C oder von maximal ± 0,02 °C ermittelt werden und beträgt günstigerweise 1 °C oder mehr, z.B. 2 °C oder mehr, 3 °C oder mehr, 4 °C oder mehr, 6 °C oder mehr, 7 °C oder mehr oder 8 °C oder mehr. Bevorzugt ist ein Bereich des Temperaturunterschieds von etwa 4°C bis etwa 40°C, besonders bevorzugt etwa 6°C bis etwa 35°C.

In einer Ausführungsform sind im Strahlengang zwischen dem zu untersuchenden Körperbereich und der Erfassungseinheit (c) optische Fokussierelemente, z.B. Linsenelemente, angeordnet, um eine möglichst punktförmige Fokussierung der in den zu untersuchenden Körperbereich eingestrahlten Terahertz-Strahlung, der reflektierten Terahertz-Strahlung und/oder der körpereigenen IR-Strahlung auf dem Sensor bzw. den Sensoren der Erfassungseinheit (c) zu ermöglichen.

So können die Sensoren der Erfassungseinheit, d.h. die IR- und/oder die Terahertz-Sensoren, z.B. der erste und/oder zweite Sensor mit optischen Fokussierelementen, z.B. Plankonvexlinsen oder Bikonvexlinsen, insbesondere Kugellinsen, aus Terahertz-transparenten Materialien wie etwa Polypropylen bzw. aus IR-transparenten Materialien wie etwa Germanium oder Zinkselenid, ausgestattet sein, wobei der Linsendurchmesser günstigerweise dem Durchmesser des Sensors angepasst ist. Durch Verwendung einer optischen Anordnung mit fokussiertem Strahlengang kann die Strahlungsausbeute und somit die Sensitivität und Präzision der Messung gesteigert werden. Besonders bevorzugt ist die Verwendung von Sensoren, z.B. Bolometern oder Thermopiles, die mit Bikonvexlinsen, insbesondere Kugellinsen, ausgestattet sind.

Die Vorrichtung wird zur Bestimmung von Glucose verwendet, die charakteristische Absorptionsbanden im Terahertz-Bereich, insbesondere in den zuvor genannten Wellenlängenregionen, z.B. in den Regionen von etwa 0,1 mm bis etwa 5 mm, von etwa 0,12 bis etwa 5 mm oder von etwa 0,5 mm bis etwa 1 mm, und im IR-Bereich, insbesondere in den zuvor genannten Wellenlängenregionen, z.B. in den Regionen von etwa 8-12 µm aufweist.

Erfindungsgemäß ist die zuvor beschriebene Vorrichtung zur nicht-invasiven Bestimmung von Glucose in Blut, insbesondere im Kapillarblut der Dermis, eingerichtet.

Die Erfindung betrifft somit die Verwendung der Vorrichtung zur nicht-invasiven Bestimmung von Glucose, insbesondere zur quantitativen Bestimmung von Glucose im Blut eines Testobjekts.

Für die Bestimmung von Glucose umfasst die Terahertz-Wellenlängenregion insbesondere einen Bereich von etwa 0,1 mm bis etwa 5 mm, von etwa 0,12 mm bis etwa 5 mm oder von etwa 0,5 bis etwa 1 mm oder einen Unterbereich davon. In diesem Bereich weist Glucose mehrere Absorptionsbänder auf, wobei drei Absorptionsbänder bei etwa 0,2 mm (entsprechend einer Wellenzahl von 50 cm⁻¹), bei etwa 0,14-0,17 mm (entsprechend einer Wellenzahl von 60-70 cm⁻¹) und bei etwa 0,13 mm (entsprechend einer Wellenzahl von 80 cm⁻¹) liegen. Vorzugsweise erfasst die Bestimmung von Glucose die Erfassung eines breitbandigen Spektrums, das mindestens 2 und günstigerweise alle 3 der o.g. Absorptionsbänder umfasst.

In der IR-Wellenlängenregion umfasst die Bestimmung von Glucose eine erste IR-Wellenlänge bzw. einen ersten IR-Wellenlängenbereich mit einem Absorptionsminimum von Glucose und eine zweite Wellenlänge bzw. einen zweiten Wellenlängenbereich mit einem Absorptionsband von Glucose oder einem Teil davon. Beispielsweise kann die erste Wellenlänge in der Region von 8,1 ± 0,3 µm und/oder 8,5 ± 0,3 µm, von 8,1 ± 0,2 µm und/oder 8,5 ± 0,2 µm oder von 8,1 ± 0,1 µm und/oder 8,5 ± 0,1 µm liegen oder zumindest eine dieser Regionen umfassen. In diesen Wellenlängenregionen weist Glucose ein Absorptionsminimum auf. Die zweite Wellenlänge kann in der Region von 9,1 ± 0,3 µm, 9,3 ± 0,3 µm und/oder 9,6 ± 0,3 µm oder von 9,1 ± 0,2 µm, 9,3 ± 0,2 µm und/oder 9,6 ± 0,2 µm oder von 9,1 ± 0,1 µm, 9,3 ± 0,1 µm und/oder 9,6 ± 0,1 µm liegen oder zumindest eine dieser Regionen umfassen. In diesen Wellenlängenregionen weist Glucose ein Absorptionsband mit mehreren Absorptionsmaxima auf.

In einer bevorzugten Ausführungsform wird die Absorption von Glucose bei zwei unterschiedlichen Wellenlängen bzw. Wellenlängenbereichen gemessen, die ein Absorptionsband von Glucose oder einen Teil davon umfassen, z.B. in der Region von 9,3 µm und in der Region von 9,6 µm.

Ein weiterer Bestandteil der Vorrichtung ist eine Einheit (f) zur temperaturkompensierten Auswertung der von der Erfassungseinheit (c) (i) und (c) (ii) stammenden Signale und zur Ermittlung der Konzentration des Analyten auf Basis der ausgewerteten Signale. Günstigerweise erfolgt eine kombinierte Auswertung der Signale aus der Terahertz-Erfassungseinheit (c) (i) und der IR-Erfassungseinheit (c) (ii). Durch kombinierte Auswertung der Signale aus dem Terahertz- und dem IR-Bereich können mehrere günstige Effekte erzielt werden. Einerseits können schwache Signale verstärkt werden, wodurch Störungen im Rahmen der Messung vermindert und eventuelle Messungenauigkeiten identifiziert und korrigiert werden können. Andererseits können Signale durch Störsubstanzen, die in einem der beiden Messbereiche Absorptionsbanden aufweisen, die mit denen des Analyten überlappen, identifiziert und eliminiert werden.

In einer bevorzugten Ausführungsform ist die Erfassungseinheit (c) für eine sequenzielle Erfassung von Terahertz-Strahlung und IR-Strahlung eingerichtet, wobei insbesondere zuerst eine Erfassung von körpereigener IR-Strahlung ohne Bestrahlung des zu untersuchenden Körperbereiches und anschließend eine Bestrahlung des zu untersuchenden Körperbereiches mit Terahertz-Strahlung und eine Erfassung der reflektierten Terahertz-Strahlung erfolgt.

Die Auswertung der Terahertz-Signale beruht darauf, dass ein breitbandiges Spektrum der Glucose über einen vorbestimmten Wellenlängenbereich in der Terahertz-Region aufgezeichnet wird, in dem sich mindestens eine Absorptionsbande, günstigerweise mindestens 2 oder 3 Absorptionsbanden der Glucose befinden. Die Intensität dieser Absorptionsbande(n) ist von der Glucose-Konzentration im Blut des Testsubjekts abhängig. Die Breite des Wellenlängenlängenbereichs beträgt günstigerweise etwa mindestens 0,05 mm oder mindestens 0,1 mm und bis zu 0,2 mm oder mehr.

Die Auswertung der IR-Signale beruht darauf, dass die körpereigene IR-Strahlung bei einer Wellenlänge oder einem Wellenlängenbereich aus einem Absorptionsminimum von Glucose, unabhängig von der im Blut des Testsubjekts vorliegenden Glucose-Konzentration ist. Die körpereigene IR-Strahlung mit einer Wellenlänge oder einem Wellenlängenbereich aus einem Absorptionsband von Glucose ist hingegen wiederum von der Glucose-Konzentration im Blut des Testsubjekts abhängig.

Auf Basis des differenziellen Signals vom ersten und zweiten IR-Sensor lässt sich - in Verbindung mit dem Signal des Terahertz-Sensors - eine ausreichend genaue Bestimmung der Glucose-Konzentration durchführen, wenn die Auswertung der Signale unter Temperaturkompensation erfolgt, insbesondere unter Berücksichtigung der durch die Elemente (d) (i), (d) (ii), (e) (i) und/oder (e) (ii) gemessenen und gegebenenfalls eingestellten Temperaturwerte für den zu untersuchenden Körperbereich und für die Sensoren der Strahlungserfassungseinheit (c). Darüber hinaus kann noch eine selektive Auswertung von IR-Strahlung erfolgen, die aus Blutgefäßen, z.B. aus Blutgefäßen der Dermis und/oder Subkutis, vorzugsweise aus Kapillarblutgefäßen der Dermis stammt.

Noch ein weiterer Bestandteil der Vorrichtung ist eine Verkleidung bzw. ein Gehäuse, das eine thermische Isolierung gegenüber der Umgebung bewirkt. Geeignete Materialien für diesen Zweck sind thermisch nicht bzw. nur gering leitfähige Kunststoffe. Dabei kann die Verkleidung bzw. das Gehäuse so ausgestaltet sein, dass der in der Aufnahmeeinheit (a) eingebrachte, zu untersuchende Körperbereich und/oder die Erfassungseinheit (c) zumindest im Wesentlichen von der Umgebung elektrisch und/oderthermisch isoliert sind, z.B. indem eine dehnbare Hülle oder Membran aus elektrisch und/oder thermisch nicht bzw. nur gering leitfähigem Material vorgesehen ist.

Die innere Oberfläche der Messvorrichtung kann außerdem mit einem für Terahertz- und/oder IR-Strahlung nicht reflektierenden und/oder einem Terahertz- und/oder IR-Strahlung absorbierenden Material vollständig oder teilweise beschichtet oder ausgestattet sein.

Ein weiterer Aspekt der Erfindung ist ein Verfahren zur nicht-invasiven quantitativen Bestimmung von Glucose im Blut eines Testsubjekts, umfassend die Schritte:
(i) Bestrahlen eines von dem Testobjekt stammenden Körperbereichs mit Terahertz-Strahlung, insbesondere in einer Wellenlängenregion von etwa 0,1 mm bis etwa 5 mm, von etwa 0,12 mm bis etwa 5 mm oder von etwa 0,1 mm bis etwa 1 mm, und Erfassen von reflektierter Terahertz-Strahlung durch eine Einheit zur Erfassung von Terahertz-Strahlung aus dem bestrahlten Körperbereich in einem Wellenlängenbereich, in dem sich die Intensität der reflektierten Terahertz-Strahlung abhängig von der Glucose-Konzentration ändert,
(ii) separates Erfassen von körpereigener IR-Strahlung aus einem von dem Testsubjekt stammenden Körperbereich durch eine Einheit zur Erfassung von IR-Strahlung bei mindestens einer ersten Wellenlänge oder einem ersten Wellenlängenbereich in der Wellenlängenregion von etwa 8 µm bis etwa 12 µm, wo die Intensität der körpereigenen IR-Strahlung von der Glucose-Konzentration im Wesentlichen unabhängig ist, und bei mindestens einer zweiten Wellenlänge oder einem zweiten Wellenlängenbereich in der Wellenlängenregion von etwa 8 µm bis etwa 12 µm, wo sich die Intensität der körpereigenen IR-Strahlung abhängig von der Glucose-Konzentration ändert, und gegebenenfalls unspezifisches Erfassen von körpereigener IR-Strahlung aus dem bestrahlten Körperoberflächenbereich zur Referenzierung,
   wobei die Temperatur im Bereich der Einheit zur Erfassung von IR-Strahlung geringer ist als die Temperatur des zu untersuchenden Körperbereichs,
(iii) kombiniertes Auswerten der gemäß (i) und (ii) erfassten Signale unter Berücksichtigung der Temperatur des zu untersuchenden Körperbereichs und der Temperatur im Bereich der Einheiten zur Erfassung von Terahertz-Strahlung und IR-Strahlung, und wobei gegebenenfalls eine selektive Auswertung von körpereigener IR-Strahlung aus Kapillarblutgefäßen der Dermis des Körperbereichs erfolgt und
(iv) Ermitteln der Glucose-Konzentration auf Basis der ausgewerteten Signale.

Die Durchführung des Verfahrens erfolgt vorzugsweise durch Erfassen von körpereigener IR-Strahlung aus einer Fingerkuppe des Testsubjekts und ohne Verwendung einer externen Quelle für IR-Strahlung, z.B. unter Verwendung der zuvor beschriebenen Vorrichtung. Die im Rahmen der Vorrichtung spezifisch offenbarten Merkmale beziehen sich ebenso auf das Verfahren.

Die Vorrichtung und das Verfahren sind zur Bestimmung von Glucose in Blut vorgesehen.

Im Folgenden wird die vorliegende Erfindung nochmals ausführlich näher erläutert. Die menschliche Haut besteht von außen nach innen aus mehreren Schichten und zwar der Oberhaut (Epidermis) mit der Hornschicht, verhornender Schicht und Keimschicht, der Lederhaut (Dermis) mit der Papillarschicht und der Netzschicht und der Unterhaut (Subkutis). Die Epidermis enthält keine Blutgefäße. Die Dermis enthält feine Kapillarblutgefäße, die mit größeren Blutgefäßen in der Subkutis in Verbindung stehen. Ein arterieller Puls liegt in dem mit Kapillargefäßen durchzogenen Bereich der Dermis, nicht jedoch in darüber liegenden Hautschichten, beispielsweise der Epidermis, vor.

Nach Bestrahlung der Hautoberfläche mit Terahertz-Strahlung wird Terahertz-Strahlung vom bestrahlten Körperbereich reflektiert, die zumindest teilweise aus dem von Kapillargefäßen durchzogenen Bereich der Dermis stammt. Auch von der Hautoberfläche ausgestrahlte IR-Strahlung, insbesondere IR-Strahlung in der Wellenlängenregion von 8-12 µm, stammt zumindest teilweise aus dem von Kapillargefäßen durchzogenen Bereich der Dermis. In diesem Bereich befindliche Substanzen mit Absorptionsbändern in der Terahertz- und IR-Region können Strahlung im Bereich dieser Absorptionsbänder absorbieren, wobei das Ausmaß der Absorption mit der Konzentration der betreffenden Substanz korreliert. Die reflektierte Terahertz-Strahlung und die körpereigene IR-Strahlung stammen aus unterschiedlichen Regionen des untersuchten Körperbereichs, wobei eine aus der Epidermis stammende Strahlung keine Abhängigkeit vom arteriellen Puls des Testsubjekts aufweist. Im Gegensatz dazu weist Strahlung, die aus dem mit Kapillargefäßen durchzogenen Bereich der Dermis stammt, ein vom arteriellen Puls des Testsubjekts abhängiges Signal auf.

Die von dem zu untersuchenden Körperbereich abgegebene Strahlung ist von der Temperatur, insbesondere der Kerntemperatur des untersuchten Körperbereichs, abhängig. Das in einem Sensor gemessene Signal der Strahlung ist daher ebenfalls von der Temperatur des untersuchten Körperbereichs, aber auch von der Temperatur des Sensors selbst und somit von der Differenz der Temperatur, insbesondere der Kerntemperatur des untersuchten Körperbereichs, und der Temperatur des zur Erfassung der Strahlung verwendeten Sensors abhängig.

Gemäß vorliegender Erfindung erfolgt daher eine temperaturkompensierte Auswertung des Messsignals, bei der die Temperatur des untersuchten Körperbereichs, die Temperatur der zur Erfassung der Terahertz- und IR-Strahlung verwendeten Sensoren und die Differenz beider Temperaturen berücksichtigt wird. Die Messung wird unter Bedingungen durchgeführt, bei denen die Temperatur des untersuchten Körperbereichs höher, beispielsweise mindestens 1 °C und vorzugsweise mindestens 6 °C höher ist als die Temperatur der zur Erfassung der körpereigenen Terahertz- und IR-Strahlung verwendeten Sensoren.

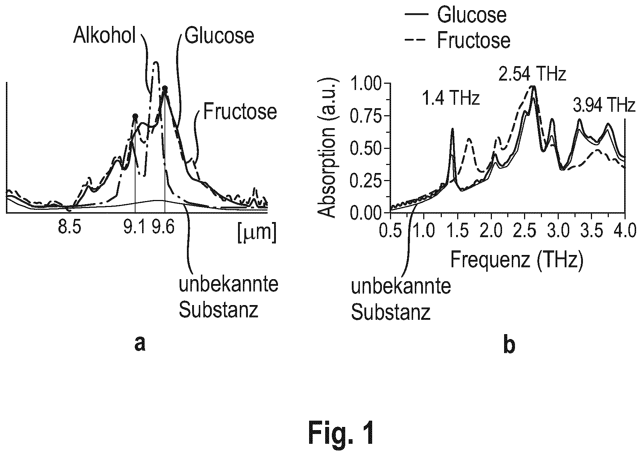

**Figur 1** zeigt schematisch Vorteile einer kombinierten Auswertung von Messsignalen aus dem Terahertz-Bereich und dem IR-Bereich, wodurch die Genauigkeit der Messung durch verbesserte Zuordnung der Messsignale zu einem spezifischen Analyten erhöht werden kann.

In Figur 1a sind die Absorptionsspektren von Alkohol (rot), Glucose (grün), Fructose (blau) und einer unbekannten Substanz (gelb) im IR-Wellenlängenbereich von etwa 8 µm bis etwa 10 µm dargestellt. Bei Verwendung von IR-Sensoren (z.B. Thermopiles) mit einer Wellenlänge von 8,5 µm (Referenz Wellenlänge) und 9,6 µm (Analyt-spezifische Wellenlänge) ist eine sichere Unterscheidung zwischen Glucose und der unbekannten Substanz möglich. Eine Unterscheidung von Glucose und Fructose ist hingegen problematisch. In Figur 1b ist ein Absorptionsspektrum im Terahertz Wellenlängenbereich von 400 µm bis 100 µm (entsprechend einer Frequenz von 0,5 bis 4,0 THz) von Glucose (schwarz), Fructose (rot; gepunktet) und der unbekannten Substanz (gelb) gezeigt. Im Terahertz-Bereich ist eine sichere Unterscheidung von Glucose und Fructose möglich, wohingegen die Absorptionsspektren von Glucose und der unbekannten Substanz überlappen. Bei Kombination der Absorptionsspektren im IR-Bereich und Terahertz-Bereich ist eine verbesserte Unterscheidung der einzelnen Substanzen möglich, wodurch eine Erhöhung der Messgenauigkeit bewirkt wird.

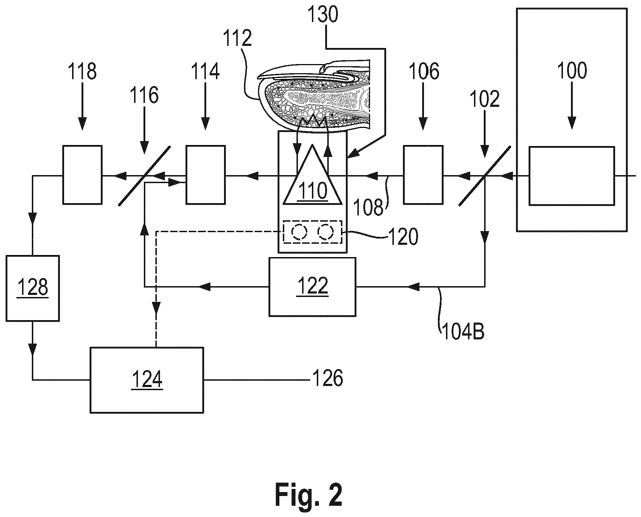

**Figur 2** zeigt eine schematische Darstellung einer Ausführungsform der Vorrichtung. Die Vorrichtung enthält eine Quelle für Terahertz-Strahlung (100), z.B. einen Femtosekunden-Laser mit einer Wellenlänge von 800 nm, dessen Strahlung zunächst durch einen semipermeablen Strahlenteiler (102) geleitet und dort in Teilstrahlen (104a) und (104b) aufgespalten wird. Teilstrahl (104a) wird in einem Mischer (106) z.B. einem Galliumarsenid-Kristall, in Terahertz-Strahlung (108) umgewandelt, die durch eine optische Vorrichtung (110), z.B. ein Prisma (auf einen zu untersuchenden Körperbereich (112), z.B. eine Fingerkuppe, geleitet wird. Die aus dem Körperbereich reflektierte Strahlung wird über das optische Element (110), einen weiteren Mischer (114), z.B. einem Galliumarsenid-Kristall, und gegebenenfalls einen weiteren semipermeablen Strahlenteiler (116) in einen Terahertz-Sensor (118), z.B. eine Photodiode, geleitet, die zur Erfassung eines Spektrum im Terahertz-Bereich eingerichtet ist. Der Teilstrahl (104b) vom Strahlenteiler (102) kann zur Referenzierung gegebenenfalls über ein Verzögerungselement (120) in den Mischer (114) und von dort in die Photodiode (118) geleitet werden. Weiterhin enthält die Vorrichtung eine IR-Erfassungseinheit (120) mit einem oder mehreren IR-Sensoren, die zur Erfassung von körpereigener IR-Strahlung aus dem zu untersuchenden Körperbereich (112) eingerichtet sind. Das Messsignal aus dem IR-Sensor (120) wird in eine Auswertungseinheit (124), z.B. eine CPU, geleitet. Die Auswertung erfolgt in der Einheit (124) in Kombination mit dem Messsignal aus dem Terahertz-Sensor (118), gegebenenfalls nach einer Fast-Fourier-Transformation in der Einheit (128). In der Auswertungseinheit (124) aus den kombinierten Terahertz- und IR-Messsignalen ein Wert (126) für die Konzentration des Analyten, z.B. Glucose ermittelt, der dann auf geeignete Weise, z.B. mittels eines Displays (nicht gezeigt) dargestellt werden kann.

Außerdem enthält die Vorrichtung ein Trägerelement (130), z.B. einen Metallträger wie etwa Kupfer, der zur Aufnahme zumindest der IR-Erfassungseinheit (120) sowie gegebenenfalls weiteren Elementen wie etwa die optische Vorrichtung (110) und/oder dem Terahertz-Sensor (118) vorgesehen ist. Die Temperatur des Trägerelements (130) kann durch geeignete Mittel stabilisiert werden, um eine temperaturkompensierte Messung des IR-Signals und gegebenenfalls des Terahertz-Signals zu ermöglichen.

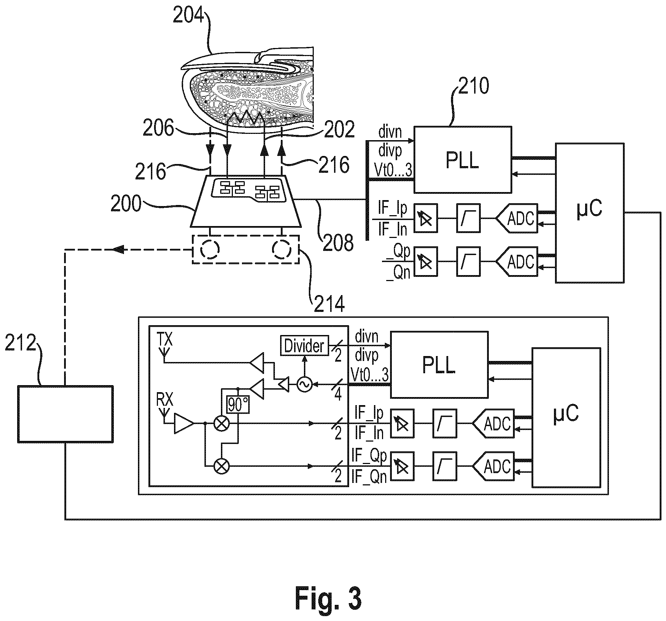

**Figur 3** zeigt eine weitere Ausführungsform der Vorrichtung. Die Vorrichtung enthält einen Terahertz-Sende-Empfangschip (200), der Terahertz-Strahlung (202) in den zu untersuchenden Körperbereich (204), z.B. eine Fingerkuppe, emittiert und zur Erfassung der davon reflektierten Terahertz-Strahlung (206) eingerichtet ist. Das Messsignal vom Chip (200) wird über eine Fast-Fourier-Transformationseinheit (210) zur Auswertungseinheit (212), z.B. einer CPU, geleitet. Weiterhin enthält die Vorrichtung mindestens einen IR-Sensor (214) zur Erfassung körpereigener IR-Strahlung (216) aus dem zu untersuchenden Körperbereich (204). Das von dem mindestens einen IR-Sensor (214) erfasste Messsignal wird in die Auswertungseinheit (212) geleitet und dort in Kombination mit dem Terahertz-Messsignal ausgewertet.

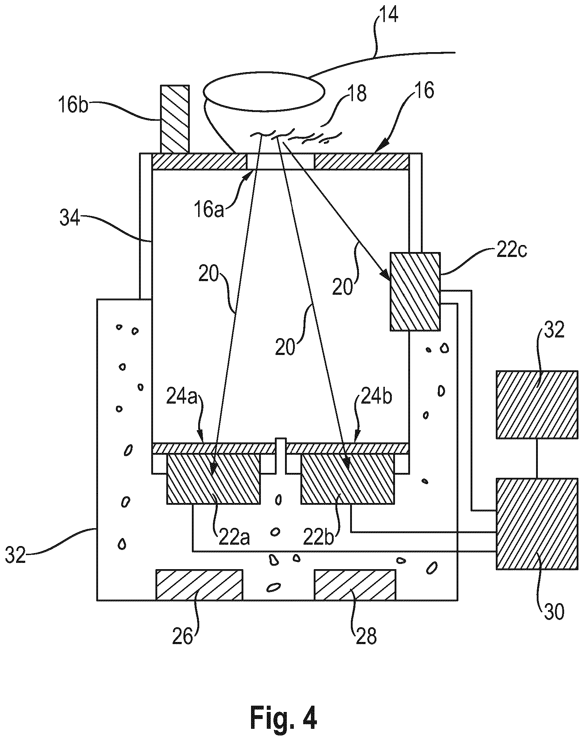

Die detaillierte Darstellung einer Ausführungsform der IR-Erfassungseinheit ist in **Figur 4** gezeigt. In die Vorrichtung wird ein zu untersuchender Körperbereich (14) eines Testsubjekts eingebracht.

Dabei wird vorzugsweise ein gut durchbluteter Körperbereich, wie etwa eine Fingerkuppe, ausgewählt. Der Körperbereich (14) liegt auf einem Auflageelement (16), welches thermisch isoliert und einen für die aus dem Körperbereich (14) stammende IR-Strahlung (20) zumindest teilweise, d.h. zumindest im Bereich der Messwellenlängen, optisch transparenten Bereich (16a) enthält. Das Auflageelement (16) enthält Mittel (16b) zur Bestimmung der Temperatur des zu untersuchenden Körperbereichs wie etwa einer Fingerkuppe (14), z.B. einen Temperatursensor. Weiterhin können Mittel, z.B. Sensoren, zur Erfassung und/oder Kontrolle der Auflageposition und/oder des Auflagedrucks des zu bestrahlenden Körperbereichs vorhanden sein (nicht gezeigt). Ferner kann das Auflageelement ein Temperatureinstellungselement enthalten (nicht gezeigt).

IR-Strahlung (20) aus dem zu untersuchenden Körperbereich (14) stammt zumindest teilweise aus den oberflächennahen Blutkapillaren im Bereich der Dermis (18), die in einem Abstand von etwa 2,5 bis 3 mm zur Körperoberfläche liegen. Ein in den Blutkapillaren oder gegebenenfalls im angrenzenden Gewebe befindlicher Analyt wird die Strahlung im Bereich seines spezifischen Absorptionsbandes absorbieren, wobei das Ausmaß der Absorption mit der Konzentration des Analyten korreliert.

Die Vorrichtung enthält weiterhin einen ersten Sensor (22a) und einen zweiten Sensor (22b) zur separaten Erfassung der körpereigenen IR-Strahlung bei unterschiedlichen Wellenlängen bzw. Wellenlängenbereichen (20) in der Region von vorzugsweise 8-12 µm. Der erste und der zweite Sensor können als Bolometer oder Thermopiles ausgebildet sein. Gegebenenfalls können der erste und der zweite Sensor auch aus Arrays von einzelnen Sensorelementen, z.B. 8 × 8 einzelnen Sensorelementen, bestehen.

Der erste Sensor (22a) ist zur selektiven Erfassung von körpereigener Strahlung mit einer ersten Wellenlänge oder einem ersten Wellenlängenbereich aus einem Absorptionsminimum von Glucose eingerichtet, wobei ein erstes Filterelement (24a) vorgesehen ist, welches für Strahlung mit der ersten Wellenlänge oder mit dem ersten Wellenlängenbereich selektiv durchlässig ist. Das heißt, das vom ersten Sensor gemessene Signal ist im Wesentlichen unabhängig von der Glucose-Konzentration. Der zweite Sensor (22b) ist wiederum zur selektiven Erfassung von körpereigener Strahlung (20) mit einer zweiten Wellenlänge oder einem zweiten Wellenlängenbereich aus einem Absorptionsband, vorzugsweise im Bereich eines Absorptionsmaximums von Glucose, eingerichtet, wobei ein zweites Filterelement (24b) vorgesehen ist, welches für Strahlung mit der zweiten Wellenlänge oder mit dem zweiten Wellenlängenbereich selektiv durchlässig ist. Dies bedeutet, dass das vom zweiten Sensor erfasste Signal abhängig von der Glucose-Konzentration ist. Gegebenenfalls können zwei oder mehr zweite Sensoren vorhanden sein, die Strahlung mit unterschiedlichen Wellenlängen oder Wellenlängenbereichen erfassen können. Für die Bestimmung von Glucose können beispielsweise zwei zweite Sensoren verwendet werden, die IR-Strahlung im Bereich von 9,1 µm und 9,6 µm jeweils separat erfassen.

Die Filterelemente (24a, 24b) sind günstigerweise so angeordnet, dass sie direkt auf den jeweiligen Sensoren (22a, 22b) aufliegen.

Gegebenenfalls enthält die Vorrichtung weiterhin einen dritten Sensor (22c), der zur unspezifischen Erfassung von körpereigener IR-Strahlung (20) eingerichtet ist und zur Referenzierung, z.B. zur Referenzierung der Körpertemperatur in dem zu untersuchenden Körperbereich (14) dient.

Die Sensoren (22a, 22b und gegebenenfalls 22c) können gegebenenfalls mit optischen Linsenelementen ausgestattet sein, z.B. Bikonvexlinsen, insbesondere Kugellinsen, um eine Fokussierung der auftreffenden körpereigenen IR-Strahlung zu ermöglichen.

Die Sensoren (22a, 22b und gegebenenfalls 22c) befinden sich in thermischer Äquilibrierung, in dem sie in Kontakt mit einem Block (32) aus leitfähigem Material, z.B. Metall, stehen bzw. darin eingebettet sind, z.B. indem sie in einer Vertiefung des Blocks angeordnet sind. Alternativ kann das thermisch leitfähige Material auch als Platte oder Folie ausgebildet sein.

Weiterhin sind Mittel (26) zur Bestimmung der Temperatur des die Sensoren (22a, 22b und gegebenenfalls 22c) enthaltenden Blocks (24), z.B. ein Temperatursensor, und Mittel (28) zur Einstellung der Temperatur des die Sensoren (22a, 22b und gegebenenfalls 22c) enthaltenden Blocks (24), z.B. Kühl- und/oder Heizelemente, vorhanden.

Die von den Sensoren (22a, 22b und gegebenenfalls 22c) stammenden Signale sowie die von den Elementen (16b) und (26) bestimmten Temperaturen werden an eine CPU-Einheit (30) übermittelt, wobei, sofern erforderlich, eine Einstellung der Temperatur für die Sensoren (22a, 22b und 22c) auf einen Wert, der geringer ist als die Temperatur des zu untersuchenden Körperbereichs, und eine temperaturkompensierte Auswertung der Signale erfolgt. Auf Basis dieser Auswertung wird die Glucose-Konzentration bestimmt. Das Ergebnis kann dann in einem Display (32) angezeigt werden.

Die Innenseite (34) des Messsystems kann vollständig oder teilweise mit einer Oberfläche aus einem Material beschichtet oder ausgestattet sein, welche die von dem Körperbereich (14) stammende IR-Strahlung (20) nicht reflektiert und/oder die von dem Körperbereich (14) stammende IR-Strahlung (20) absorbiert.

Das Messsystem kann weiterhin eine Verkleidung oder ein Gehäuse aufweisen, das eine elektrische und/oder thermische Isolierung gegenüber der Umgebung bewirkt.

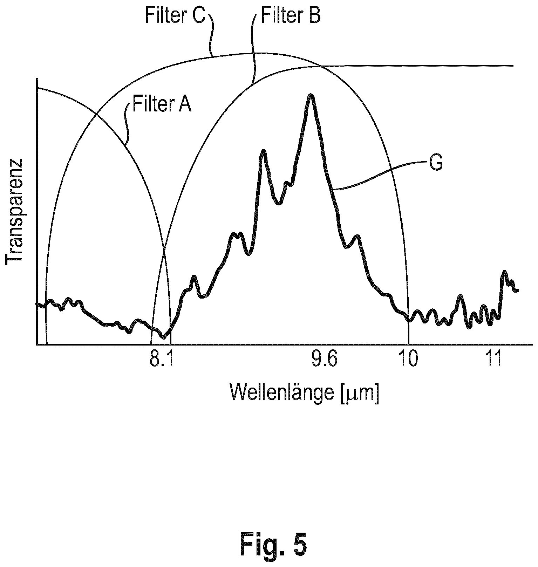

Eine alternative Ausführungsform zur separaten Erfassung von körpereigener IR-Strahlung bei unterschiedlichen Wellenlängen ist in **Figur 5** gezeigt. Die Absorptionskurve von Glucose in der Region zwischen 8 und 14 µm ist als fett gezeichnete Linie (G) dargestellt. Zur separaten Erfassung von IR-Strahlung mit zwei unterschiedlichen Wellenlängen aus dieser Region werden zwei Sensoren verwendet, die unterschiedliche Kombinationen von Bandpass-, Hochpass- und/oder Tiefpassfilterelementen umfassen. In einer Ausgestaltung enthalten beide Sensoren ein weites Bandpassfilter (C) mit einer Durchlässigkeit in der Region zwischen 8 und 14 µm. Beim ersten Sensor wird Filter (C) mit einem Hochpassfilter (A) kombiniert, der für IR-Strahlung mit einer Wellenlänge von etwa 8,5 µm oder weniger durchlässig ist. Ein Sensor, der mit den Filtern (A) und (C) ausgestattet ist, wird daher ein Signal aus der Region von 8-8,5 µm erfassen, das von der Konzentration der Glucose im Wesentlichen unabhängig ist. Der zweite Sensor ist mit einer Kombination des Bandpassfilters (C) mit einem Tiefpassfilter (B) ausgestattet, der für IR-Strahlung mit einer Wellenlänge von 8,5 µm oder höher durchlässig ist. Das mit diesem Sensor erfasste Signal umfasst ein in der Region von etwa 9-10 µm lokalisiertes Absorptionsband von Glucose und ist daher von der Glucosekonzentration abhängig. Durch differenzielle Auswertung der von beiden Sensoren erfassten Signale kann die Glucosekonzentration bestimmt werden.

In einer anderen Ausgestaltung kann auch ein erster Sensor mit dem Bandpassfilter (C) und dem Hochpassfilter (A) ausgestattet sein, während ein zweiter Sensor nur mit dem Bandpassfilter (C) ausgestattet ist. Das mit dem ersten Sensor erfasste Signal ist von der Glucosekonzentration unabhängig, während das vom zweiten Sensor erfasste Signal sich mit der Glucosekonzentration ändert.

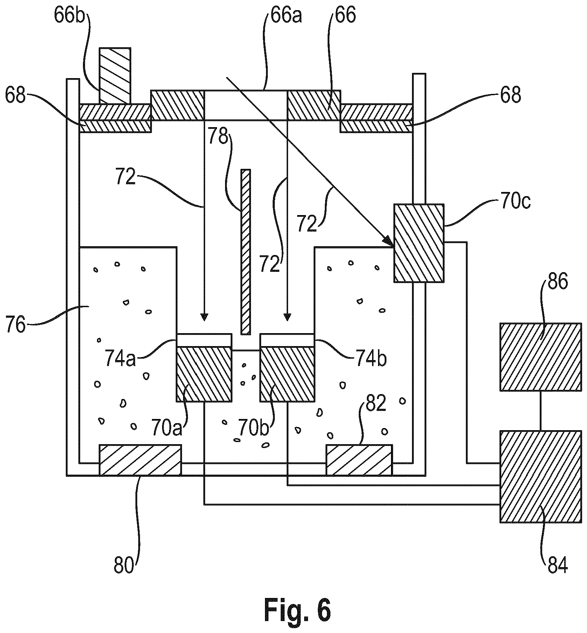

**Figur 6** zeigt die schematische Darstellung des Querschnitts einer weiteren Ausführungsform der IR-Erfassungseinheit der Vorrichtung. Dabei ist der zu untersuchende Körperbereich des Testsubjekts (nicht gezeigt) auf einem thermisch isolierenden Auflageelement (66) angeordnet, welches einen für IR-Strahlung zumindest teilweise optisch transparenten Bereich (66a) enthält. Beispielsweise handelt es sich bei dem optisch transparenten Bereich (66a) um eine Si- oder Ge-Scheibe. Das Auflageelement (66) ist mit einem Temperatursensor (66b) und gegebenenfalls mit einem TemperaturEinstellungselement (nicht gezeigt) versehen, welches vorzugsweise ein Peltier-Element umfasst. Die Temperatur des Körperbereichs kann dabei beispielsweise auf einen Bereich von etwa 28-38 °C eingestellt werden. Vorzugsweise enthält das Auflageelement (66) Mittel, z.B. Sensoren (68), zur Erfassung und/oder Kontrolle des Auflagedrucks, wie etwa eine Wägezelle, sowie gegebenenfalls Sensoren zur Erfassung und/oder Kontrolle der Auflageposition, wie etwa eine Kamera und/oder einen Pulssensor. Günstigerweise wird ein Auflagedruck von etwa 1-50 N, z.B. etwa 20 N eingestellt.

Die Vorrichtung enthält weiterhin einen ersten IR-Sensor (70a) und einen zweiten IR-Sensor (70b) zur Erfassung der körpereigenen IR-Strahlung (72) mit unterschiedlichen Wellenlängen in der Region von 0,7-20 µm, vorzugsweise von 3-20 µm aus dem untersuchten Körperbereich. Der erste und der zweite Sensor können jeweils als Bolometer oder als Thermopile ausgebildet sein. Gegebenenfalls können der erste und der zweite Sensor auch aus Arrays von einzelnen Sensorelementen bestehen.

Der erste Sensor (70a) kann zur selektiven Erfassung von körpereigener IR-Strahlung mit einer ersten Wellenlänge aus einem Absorptionsminimum des Analyten eingerichtet sein, beispielsweise, wobei ein erstes Filterelement (74a), z.B. ein Bandpassfilter mit enger Durchlässigkeit vorgesehen ist, welches für Strahlung mit der ersten Wellenlänge selektiv durchlässig ist, bei der das Signal im Wesentlichen unabhängig von der Konzentration des zu bestimmenden Analyten ist. Für die Bestimmung von Glucose liegt die erste Wellenlänge beispielsweise bei 8,1 ± 0,3 µm und/oder 8,5 ± 0,3 µm vorzugsweise bei 8,1 ± 0,2 µm und/oder 8,5 ± 0,2 µm, besonders bevorzugt bei 8,1 ± 0,2 µm und/oder 8,5 ± 0,1 µm. Der zweite Sensor (74b) ist wiederum zur selektiven Erfassung von körpereigener IR-Strahlung (72) mit einer zweiten Wellenlänge aus einem Absorptionsband, vorzugsweise im Bereich eines Absorptionsmaximums des zu bestimmenden Analyten, eingerichtet, wobei ein zweites Filterelement (74b), z.B. ein Bandpassfilter mit enger Durchlässigkeit, vorgesehen ist, welches für Strahlung mit der zweiten Wellenlänge selektiv durchlässig ist. Bei der Bestimmung von Glucose liegt die zweite Wellenlänge beispielsweise bei 9,1 ± 0,3 µm, 9,3 ± 0,3 µm und/oder 9,6 ± 0,3 µm, vorzugsweise bei 9,1 ± 0,2 µm, 9,3 ± 0,2 µm und/oder 9,6 ± 0,2 µm, besonders bevorzugt im Bereich von 9,1 ± 0,1 µm, 9,3 ± 0,1 µm und/oder 9,6 ± 0,1 µm. Gegebenenfalls können zwei oder mehr zweite Sensoren vorhanden sein, die Strahlung mit unterschiedlichen Wellenlängen oder Wellenlängenbereichen erfassen können. Für die Bestimmung von Glucose können beispielsweise zwei zweite Sensoren verwendet werden, die IR-Strahlung im Bereich von 9,1 µm oder 9,3 µm und 9,6 µm jeweils separat erfassen.

Die Filterelemente (74a, 74b) stehen günstigerweise in direktem Kontakt mit den Sensoren (70a, 70b).

Alternativ kann anstelle von Bandpassfiltern mit enger Durchlässigkeit auch die in **Figur 5** dargestellte Kombination eines Bandpassfilters mit weiter Durchlässigkeit sowie einem Hochpass- und/oder einem Tiefpassfilter verwendet werden.

Im Strahlengang der körpereigenen IR-Strahlung (72) zwischen dem zu untersuchenden Körperteil und den Sensoren (70a, 70b und gegebenenfalls 70c) können optische Fokussierelemente, z.B. Linsenelemente, angeordnet sein. So können die Sensoren (70a, 70b) gegebenenfalls mit optischen Linsenelementen ausgestattet sein, z.B. Bikonvexlinsen, insbesondere Kugellinsen, um eine Fokussierung der auftreffenden körpereigenen IR-Strahlung zu ermöglichen.

Gegebenenfalls enthält die Vorrichtung weiterhin einen dritten Sensor (70c), der zur unspezifischen Erfassung von körpereigener IR-Strahlung (72) eingerichtet ist und zur Referenzierung, z.B. zur Referenzierung der Körpertemperatur des zu untersuchenden Körperbereichs dient. Der dritte Sensor (70c) kann als Bolometer oder Thermopile ausgebildet sein.

Zwischen dem ersten Sensor (70a) und dem zweiten Sensor (70b) kann gegebenenfalls eine Zwischenwand (78) angeordnet sein.

Die Sensoren (70a, 70b und gegebenenfalls 70c) befinden sich in thermischer Äquilibrierung, indem sie in Kontakt mit thermisch leitfähigem Material, z.B. einem Block (76), einer Platte oder einer Folie aus Metall, stehen.

Weiterhin ist ein Element (80) zur Bestimmung der Temperatur des die Sensoren (70a, 70b und 70c) enthaltenden Blocks (76) vorhanden, z.B. ein Temperatursensor. Weiterhin ist ein Element (82) zur Einstellung der Temperatur des die Sensoren (70a, 70b und 70c) enthaltenden Blocks (76) vorgesehen, z.B. ein Kühl- und/oder Heizelement.

Die von den Sensoren (70a, 70b und gegebenenfalls 70c) stammenden Signale sowie die von den Elementen (66b) und (80) bestimmten Temperaturen werden an eine CPU-Einheit (84) zur temperaturkompensierten Auswertung der Signale übermittelt. Auf Basis dieser Auswertung wird die Konzentration des Analyten bestimmt, das Ergebnis kann dann in einem Display (86) gezeigt werden. Die CPU-Einheit kann außerdem zur Steuerung der Elemente (68, 82) verwendet werden.

Gegebenenfalls kann die innere Oberfläche des Messsystems mit einem für IR-Strahlung nicht reflektierenden und/oder einem IR-Strahlung absorbierenden Material beschichtet sein. Weiterhin kann das Messsystem eine thermische Isolierung gegenüber der Umgebung aufweisen.

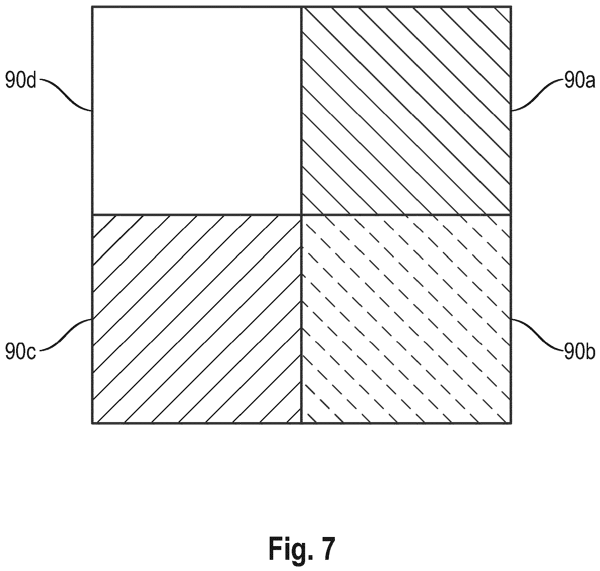

Eine besonders bevorzugte Anordnung von Sensoren in der IR-Erfassungseinheit ist in **Figur 7** gezeigt. Dabei sind vier IR-Sensoren (90a, 90b, 90c und 90d) vorhanden, die gemeinsam in einer Vertiefung eines Metallblocks (nicht gezeigt) angeordnet und damit thermisch äquilibriert sind. Vorzugsweise besteht jeder der Sensoren aus einem Array mehrerer, z.B. 8 × 8 einzelner Sensorelemente. Die Sensoren (90a, 90b) sind zur selektiven Erfassung von IR-Strahlung mit einer Wellenlänge aus einem Absorptionsband der zu bestimmenden Analyten vorgesehen und sind daher mit entsprechenden Filterelementen ausgestattet. Zur Bestimmung von Glucose kann der Sensor (90a) zur selektiven Erfassung von IR-Strahlung mit einer Wellenlänge von etwa 9,6 µm, z.B. 9,6 ± 0,1 µm und der Sensor (90b) zur selektiven Erfassung von IR-Strahlung mit einer Wellenlänge von etwa 9,1 µm, z.B. 9,1 ± 0,1 µm oder etwa 9,3 µm, z.B. 9,3 ± 0,1 µm vorgesehen sein. Der Sensor (90c) ist zur selektiven Erfassung von IR-Strahlung mit einer Wellenlänge aus einem Absorptionsminimum der zu bestimmenden Analyten vorgesehen und sind daher mit entsprechenden Filterelementen ausgestattet. Zur Bestimmung von Glucose kann der Sensor (90c) zur selektiven Erfassung von IR-Strahlung mit einer Wellenlänge von etwa 8,5 µm, z.B. 8,5 ± 0,1 µm, vorgesehen sein. Der Sensor (90d) enthält kein Filterelement und dient zur Referenzierung.

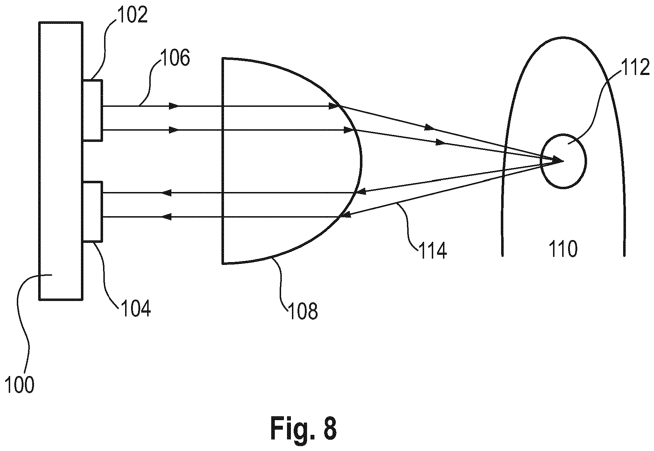

Eine besonders bevorzugte Ausführungsform eines Terahertz-Sende/Empfangselements mit einer Fokussierungslinse ist in **Figur 8** gezeigt. Ein Terahertz-Sende/Empfangselement (100) enthält eine Terahertz-Strahlungsquelle (102), z.B. eine Antenne, und eine Terahertz-Erfassungseinheit (104).

Von der Strahlungsquelle (102) emittierte Terahertz-Strahlung (106) wird durch eine Fokussierungslinse (108), z.B. eine sphärische Linse, geleitet. Die Linse besteht aus einem für Terahertz-Strahlung transparentem Material wie Polypropylen.

Die Strahlung (106) wird durch die Linse (108) auf eine vorbestimmte Zone (112) des zu untersuchenden Körperbereichs (110) z.B. eines Fingers (Kapillarblutbereich oder aufgesättigtes Gewebe), fokussiert. Vorzugsweise erfolgt die Fokussierung auf eine vorbestimmte Eindringtiefe, z.B. auf eine Eindringtiefe von 3-4 mm. Die von der Fokussierungszone (112) reflektierte Strahlung (114) wird wiederum durch die Linse (108) geleitet, wobei eine Fokussierung auf die Terahertz-Erfassungseinheit (104) erfolgt.

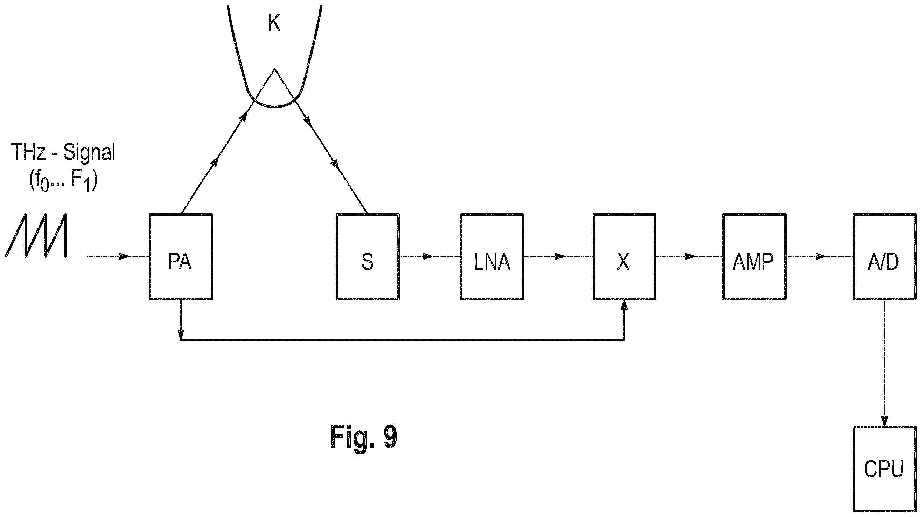

In **Figur 9** ist eine besonders bevorzugte Ausführungsform für die Auswertung eines Terahertz-Messsignals gezeigt. Ein Terahertz-Signal wird in seiner Frequenz über einen Bereich (f0...f1) moduliert, der eine Absorptionsbande von Glucose als dem zu bestimmenden Analyten enthält. Die Modulation kann kontinuierlich, z.B durch Spannungsänderung mit einem spannungsgesteuertem Oszillator (VCO), oder diskontinuierlich erfolgen.

Das frequenzmodulierte Terahertz-Signal gelangt an eine Antenne, z.B. an eine Patch-Antenne (PA) und wird dort an den zu untersuchenden Körperbereich (K), z.B. einen Finger, gestrahlt. Dies kann über eine Linse (siehe z.B. **Figur 8**) oder einen Hohlleiter erfolgen. Dort wird die Terahertz-Strahlung reflektiert und gelangt zu einem Sensor (S), z.B. an eine Patch-Antenne oder einen Dipol und von dort an einen Vorverstärker, z.B. an einen LNA.

Danach wird das Signal zu einem Mischer (X) geleitet, der das Empfangssignal mit dem Sendesignal multipliziert. Das Empfangssignal hat aufgrund der Wegstrecke zum Körperbereich und wieder zurück eine unterschiedliche Weglänge wie das Sendesignal. Aufgrund der Frequenzmodulation und der unterschiedlichen Weglänge und Laufzeit für das Empfangssignal und das Sendesignal, ergibt sich eine zum Laufzeitunterschied proportionale Frequenzdifferenz (z.B. Weglängen-Unterschied ca. 50 mm entsprechend einem Laufzeitunterschied von 166 psec) am Mischer. Dadurch entsteht ein gemischtes Signal (sin(fa)*(fb) = sin(fa+fb) und sin(fa-fb), wobei die Differenz das Nutzsignal ist, das durch einen Verstärker (AMP) weiter verstärkt wird. Eine oder mehrere Phasen des Signals werden zu einem A/D-Wandler geleitet und durch eine CPU ausgewertet.

Die Auswertung umfasst vorzugsweise eine laufend durchgeführte Fast Fourier-Transformation (FFT), um unerwünschte Störsignale wegzufiltern. So entsteht bei zunehmender Frequenz (f0...f1) durch den Spannungseingang gesteuert am THz-Sender jeweils ein unterschiedliches Spektrum. Das Signal, welches dem Strahlengang zur Kapillarschicht und zurück entspricht, kann über seinen charakteristischen Laufzeitunterschied identifiziert und abgegriffen werden. Dadurch wird ein charakteristischer Kurvenverlauf erhalten, der dem Reflexions- und Absorptionsspektrum von Glucose im Kapillarblut entspricht. Eine Vergleichsmessung bei den Absorptionsmaxima und den Absorptionsminima von Glucose ergibt den Glucosegehalt im Kapillarblut.

Alternativ zur Frequenzmodulation kann das Terahertz-Signal auch als pulsmoduliertes Signal auf den zu untersuchenden Körperbereich gestrahlt werden, wobei in vorbestimmten Zeitabständen Signalpulse, insbesondere diskrete Signalpulse mit jeweils unterschiedlicher Frequenz eingestrahlt werden.

## Patentansprüche

1. Verwendung einer Vorrichtung zur nicht-invasiven Bestimmung von Glucose im Blut eines Testsubjekts, wobei die Vorrichtung umfasst:
(a) eine Einheit (16) zur Aufnahme eines von dem Testsubjekt stammenden und zu untersuchenden Körperbereichs (14),
(b) eine Strahlungsquelle (100) zur Erzeugung von Terahertz-Strahlung insbesondere von Terahertz-Strahlung in einer Wellenlängenregion von etwa 0,1 mm bis etwa 5 mm, von etwa 0,12 mm bis etwa 5 mm oder von etwa 0,1 mm bis etwa 1 mm, zur Bestrahlung des zu untersuchenden Körperbereichs,
(c) eine Einheit zur Erfassung von Strahlung aus dem zu untersuchenden Körperbereich, umfassend:
(i) eine Einheit (118) zur Erfassung von reflektierter Terahertz-Strahlung aus dem zu untersuchenden Körperbereich, die zur Erfassung von Terahertz-Strahlung in einem Wellenlängenbereich eingerichtet ist, in dem sich die Intensität der reflektierten Terahertz-Strahlung abhängig von der Glucose-Konzentration ändert,
(ii) eine Einheit (120) zur Erfassung von körpereigener IR-Strahlung aus dem zu untersuchenden Körperbereich, die zur separaten Erfassung von IR-Strahlung bei mindestens zwei unterschiedlichen Wellenlängen oder Wellenlängenbereichen in der Wellenlängenregion von etwa 8 µm bis etwa 12 µm eingerichtet ist,
wobei bei einer ersten Wellenlänge oder einem ersten Wellenlängenbereich die Intensität der körpereigenen IR-Strahlung von der Glucose-Konzentration im Wesentlichen unabhängig ist, und
wobei bei einer zweiten Wellenlänge oder einem zweiten Wellenlängenbereich sich die Intensität der körpereigenen IR-Strahlung abhängig von der Glucose-Konzentration ändert und
wobei die Einheit gegebenenfalls zusätzlich zur unspezifischen Erfassung von körpereigener IR-Strahlung eingerichtet ist, und
(d)
(i) ein Element (16b) zur Messung der Temperatur in dem zu untersuchenden Körperbereich,
(ii) gegebenenfalls ein Element zur Einstellung der Temperatur in dem zu untersuchenden Körperbereich,
(e)
(i) ein Element (26) zur Messung der Temperatur in der Erfassungseinheit (c),
(ii) ein Element (28) zur Einstellung der Temperatur in der Erfassungseinheit (c),
wobei vorgesehen ist, dass die Temperatur in der Erfassungseinheit (c) geringer ist als die Temperatur in dem zu untersuchenden Körperbereich, und
(f) eine Einheit (124), die zur temperaturkompensierten Auswertung der von der Erfassungseinheit (c) (i) stammenden Terahertz-Signale und von der Erfassungseinheit (c) (ii) stammenden körpereigenen IR-Signale und zur Ermittlung der Glucose-Konzentration auf Basis der ausgewerteten Terahertz-Signale und körpereigenen IR-Signale eingerichtet ist, wobei die Einheit gegebenenfalls zur selektiven Auswertung von körpereigener IR-Strahlung eingerichtet ist, die aus Kapillarblutgefäßen der Dermis des Körperbereichs stammt.

2. Verwendung nach Anspruch 1,
**dadurch gekennzeichnet, dass** die Strahlungsquelle zur Erzeugung von Terahertz-Strahlung (b) eine Antenne, insbesondere eine Patch- oder eine Dipol-Antenne umfasst, und vorzugsweise
eingerichtet ist, eine frequenzmodulierte oder pulsmodulierte Terahertz-Strahlung auszustrahlen.

3. Verwendung nach Anspruch 1 oder 2,
**dadurch gekennzeichnet, dass** im Strahlengang zwischen der Strahlungsquelle zur Erzeugung von Terahertz-Strahlung (b) und dem zu untersuchenden Körperbereich eine Fokussierungslinse, z.B. eine sphärische Linse, eine asphärische Linse oder eine Fresnel-Linse angeordnet ist, wobei die Fokussierungslinse aus einem für Terahertz-Strahlung im Wesentlichen transparenten Material besteht, wobei
die Fokussierungslinse vorzugsweise eingerichtet ist, die von der Terahertz-Strahlungsquelle (b) erzeugte Strahlung auf eine vorbestimmte Zone des zu untersuchenden Körperbereichs, insbesondere auf eine Kapillarblut enthaltende Zone zu fokussieren.

4. Verwendung nach einem der Ansprüche 1-3,
**dadurch gekennzeichnet, dass** die Einheit (c) (i) eingerichtet ist, ein breitbandiges Spektrum im Terahertz-Bereich, insbesondere innerhalb eines Frequenzbereichs von etwa 0,12 mm bis etwa 5 mm (entsprechend 60 GHz bis etwa 2.5 THz), zu erfassen, und/oder
dass die Einheit (c) (i) eingerichtet ist, eine ein- oder mehrstufige Verstärkung, insbesondere eine zweistufige Verstärkung der vom zu untersuchenden Körperbereich reflektierten Terahertz-Strahlung zu bewirken.

5. Verwendung nach einem der Ansprüche 1-4,
**dadurch gekennzeichnet, dass** die Einheit (c) (i) zur kombinierten Erfassung von reflektierter Terahertz-Strahlung aus dem zu untersuchenden Körperbereich und in den zu untersuchenden Körperbereich eingestrahlter Terahertz-Strahlung eingerichtet ist, und wobei die Einheit (c) (i) insbesondere zur Erfassung von frequenzmodulierten oder pulsmodulierten Signalen eingerichtet ist.

6. Verwendung nach einem der Ansprüche 1-5,
**dadurch gekennzeichnet,**
**dass** die Vorrichtung keine externe Quelle zur Erzeugung von IR-Strahlung enthält.

7. Verwendung nach einem der Ansprüche 1-6,
dass die Einheit zur Aufnahme des zu untersuchenden Körperbereichs (a) ein gegenüber der Einheit (c) zur Erfassung von Strahlung zumindest teilweise thermisch isolierendes Element zur Auflage des zu untersuchenden Körperbereichs umfasst, das einen für Terahertz-Strahlung insbesondere von etwa 0,12 mm bis etwa 5 mm (entsprechend von etwa 60 GHz bis etwa 2.5 THz) oder in einem Unterbereich davon und für IR-Strahlung in der Wellenlängenregion insbesondere von etwa 8 µm bis 12 µm oder in einem Unterbereich davon transparenten Bereich enthält.

8. Verwendung nach einem der Ansprüche 1-7,
**dadurch gekennzeichnet,**
**dass** die Einheit zur Aufnahme des zu untersuchenden Körperflächenbereichs (a) Mittel, z.B. Sensoren, zur Erfassung und/oder Kontrolle von Auflageposition und/oder Auflagedruck des zu untersuchenden Körperbereichs umfasst, wobei insbesondere Mittel zur Erfassung und/oder Kontrolle eines Auflagedrucks im Bereich von etwa 0,5-100 N, vorzugsweise im Bereich von etwa 1-50 N und besonders bevorzugt von etwa 20 N vorgesehen sind.

9. Verwendung nach einem der Ansprüche 1-8,
**dadurch gekennzeichnet**
**dass** die Einheit zur Erfassung von körpereigener IR-Strahlung (c) (ii) mindestens einen ersten Sensor, mindestens einen zweiten Sensor und gegebenenfalls mindestens einen dritten Sensor umfasst,
wobei der erste Sensor zur Erfassung von IR-Strahlung mit einer ersten Wellenlänge oder einem ersten Wellenlängenbereich in der Region von etwa 8 µm bis etwa 12 µm eingerichtet ist, wo die Intensität der körpereigenen IR-Strahlung von der Glucose-Konzentration im Wesentlichen unabhängig ist,
wobei der zweite Sensor zur Erfassung von IR-Strahlung mit einer zweiten Wellenlänge oder einem zweiten Wellenlängenbereich in der Region von etwa 8 µm bis etwa 12 µm eingerichtet ist, wo sich die Intensität der körpereigenen IR-Strahlung abhängig von der Glucose-Konzentration ändert, und
wobei der dritte Sensor zur Referenzierung der körpereigenen IR-Strahlung eingerichtet ist, wobei
die Einheit zur Erfassung von körpereigener IR-Strahlung (c) (ii) vorzugsweise zwei oder mehr zweite Sensoren umfasst, die zur Erfassung von IR-Strahlung bei unterschiedlichen Wellenlängen oder Wellenlängenbereichen eingerichtet sind, wo sich die Intensität der körpereigenen IR-Strahlung abhängig von der Glucose-Konzentration ändert.

10. Verwendung nach einem der Ansprüche 1-9,
**dadurch gekennzeichnet,**
**dass** die Einheit zur Erfassung von Strahlung (c) in Kontakt mit einem wärmeleitfähigen Träger steht, wobei insbesondere die Einheit zur Erfassung von Terahertz-Strahlung (c) (i) und die Einheit zur Erfassung von IR-Strahlung (c) (ii) in Kontakt mit dem gleichen wärmeleitfähigen Träger stehen.

11. Verwendung nach einem der Ansprüche 1-10,
**dadurch gekennzeichnet,**
**dass** eine Temperatur in der Erfassungseinheit (c) vorgesehen ist, die um mindestens 5°C, um mindestens 6°C oder um mindestens 7°C und um bis zu 15°C geringer ist als die Temperatur des zu untersuchenden Körperbereichs.

12. Verwendung nach einem der Ansprüche 1-11,
**dadurch gekennzeichnet, dass** die Vorrichtung für eine sequenzielle Erfassung von Terahertz-Strahlung und IR-Strahlung vorgesehen ist, wobei insbesondere zuerst eine Erfassung von körpereigener IR-Strahlung ohne Bestrahlung des zu untersuchenden Körperbereiches und anschließend eine Bestrahlung des zu untersuchenden Körperbereiches mit Terahertz-Strahlung und eine Erfassung der reflektierten Terahertz-Strahlung erfolgt.

13. Verwendung nach einem der Ansprüche 1-12,
**dadurch gekennzeichnet,**
**dass** die Einheit (f) zur temperaturkompensierten Auswertung der Signale auf Basis der durch die Elemente (d) und/oder (e) gemessenen und gegebenenfalls eingestellten Temperaturwerte vorgesehen ist, und/oder
**dass** die Einheit (f) zur kombinierten Auswertung der Signale aus der Terahertz-Erfassungseinheit (c) (i) und der IR-Erfassungseinheit (c) (ii) vorgesehen ist.

14. Verwendung nach einem der Ansprüche 1-13 zur nicht-invasiven quantitativen Bestimmung von Glucose im Blut eines Testsubjekts.

15. Verfahren zur nicht-invasiven quantitativen Bestimmung von Glucose im Blut eines Testsubjekts, umfassend die Schritte:
(i) Bestrahlen eines von dem Testobjekt stammenden Körperbereichs mit Terahertz-Strahlung, insbesondere in einer Wellenlängenregion von etwa 0,1 mm bis etwa 5 mm, von etwa 0,12 mm bis etwa 5 mm oder von etwa 0,1 mm bis etwa 1 mm, und Erfassen von reflektierter Terahertz-Strahlung durch eine Einheit zur Erfassung von Terahertz-Strahlung aus dem bestrahlten Körperbereich in einem Wellenlängenbereich, in dem sich die Intensität der reflektierten Terahertz-Strahlung abhängig von der Glucose-Konzentration ändert,
(ii) separates Erfassen von körpereigener IR-Strahlung aus einem von dem Testsubjekt stammenden Körperbereich durch eine Einheit zur Erfassung von IR-Strahlung bei mindestens einer ersten Wellenlänge oder einem ersten Wellenlängenbereich in der Wellenlängenregion von etwa 8 µm bis etwa 12 µm, wo die Intensität der körpereigenen IR-Strahlung von der Glucose-Konzentration im Wesentlichen unabhängig ist, und bei mindestens einer zweiten Wellenlänge oder einem zweiten Wellenlängenbereich in der Wellenlängenregion von etwa 8 µm bis etwa 12 µm, wo sich die Intensität der körpereigenen IR-Strahlung abhängig von der Glucose-Konzentration ändert, und gegebenenfalls unspezifisches Erfassen von körpereigener IR-Strahlung aus dem bestrahlten Körperoberflächenbereich zur Referenzierung,
wobei die Temperatur im Bereich der Einheit zur Erfassung von IR-Strahlung geringer ist als die Temperatur des zu untersuchenden Körperbereichs,
(iii) kombiniertes Auswerten der gemäß (i) und (ii) erfassten Signale unter Berücksichtigung der Temperatur des zu untersuchenden Körperbereichs und der Temperatur im Bereich der Einheiten zur Erfassung von Terahertz-Strahlung und IR-Strahlung, und wobei gegebenenfalls eine selektive Auswertung von körpereigener IR-Strahlung aus Kapillarblutgefäßen der Dermis des Körperbereichs erfolgt und
(iv) Ermitteln der Glucose-Konzentration auf Basis der ausgewerteten Signale,
wobei die Bestimmung der Glucose vorzugsweise durch Erfassen von körpereigener IR-Strahlung aus einer Fingerkuppe des Testsubjekts ohne Anregung durch eine externe Quelle von IR-Strahlung erfolgt.

## Claims

1. Use of a device for the non-invasive determination of glucose in the blood of a test subject, wherein the device comprises:
(a) a unit (16) for receiving a body region (14) originating from the test subject and that is to be investigated,
(b) a radiation source (100) for generating terahertz radiation, in particular terahertz radiation in a wavelength region from about 0.1 mm to about 5 mm, from about 0.12 mm to about 5 mm or from about 0.1 mm to about 1 mm, for irradiating the body region to be investigated,
(c) a unit for detecting radiation from the body region to be investigated, comprising:
(i) a unit (118) for detecting reflected terahertz radiation from the body region to be investigated, which is adapted to detect terahertz radiation in a wavelength range in which the intensity of the reflected terahertz radiation changes depending on the glucose concentration,
(ii) a unit (120) for detecting the body's own IR radiation from the body region to be investigated, which is adapted for the separate detection of IR radiation at at least two different wavelengths or wavelength ranges in the wavelength region from about 8 µm to about 12 µm,
wherein at a first wavelength or a first wavelength range the intensity of the body's own IR radiation is substantially independent of the glucose concentration, and
wherein at a second wavelength or a second wavelength range the intensity of the body's own IR radiation changes depending on the glucose concentration and
wherein the unit is optionally additionally adapted for the non-specific detection of the body's own IR radiation, and
(d)
(i) an element (16b) for measuring the temperature in the body region to be investigated,
(ii) optionally an element for setting the temperature in the body region to be investigated,
(e)
(i) an element (26) for measuring the temperature in the detection unit (c),
(ii) an element (28) for setting the temperature in the detection unit (c),
wherein it is provided that the temperature in the detection unit (c) is lower than the temperature in the body region to be investigated, and
(f) a unit (124) which is adapted for the temperature-compensated evaluation of the terahertz signals originating from the detection unit (c) (i) and the body's own IR signals originating from the detection unit (c) (ii) and for the determination of the glucose concentration on the basis of the evaluated terahertz signals and the body's own IR signals,
wherein the unit is optionally adapted for the selective evaluation of the body's own IR radiation originating from capillary blood vessels of the dermis of the body region.

2. Use according to claim 1,
**characterised in that** the radiation source for generating terahertz radiation (b) comprises an antenna, in particular a patch or a dipole antenna, and is preferably designed to emit frequency-modulated or pulse-modulated terahertz radiation.

3. Use according to claim 1 or 2,
**characterised in that** a focusing lens, e.g. a spherical lens, an aspherical lens or a Fresnel lens, is arranged in the beam path between the radiation source for generating terahertz radiation (b) and the body region to be investigated, wherein the focusing lens is made of a material that is substantially transparent to terahertz radiation, wherein the focusing lens is preferably designed to focus the radiation generated by the terahertz radiation source (b) onto a predetermined zone of the body region to be investigated, in particular onto a zone containing capillary blood.

4. Use according to one of claims 1-3,
**characterised in that** the unit (c) (i) is adapted to detect a broadband spectrum in the terahertz range, in particular within a frequency range from about 0.12 mm to about 5 mm (corresponding to 60 GHz to about 2.5 THz), and/or
that the unit (c) (i) is adapted to perform a single-stage or multi-stage amplification, in particular a two-stage amplification, of the terahertz radiation reflected from the body region to be investigated.

5. Use according to one of claims 1-4,
**characterised in that** the unit (c) (i) is adapted for the combined detection of reflected terahertz radiation from the body region to be investigated and terahertz radiation radiated into the body region to be investigated, and wherein the unit (c) (i) is adapted in particular for the detection of frequency-modulated or pulse-modulated signals.

6. Use according to one of claims 1-5,
**characterised in that** the device does not contain an external source for generating IR radiation.

7. Use according to one of claims 1-6,
**characterised in that** that the unit for recording the body region to be investigated (a) comprises an element for supporting the body region to be investigated that is at least partially thermally insulating with respect to the unit (c) for detecting radiation, and that contains a region that is transparent to terahertz radiation, in particular from about 0.12 mm to about 5 mm (corresponding from about 60 GHz to about 2.5 THz) or in a sub-range thereof and to IR radiation in the wavelength region in particular from about 8 µm to 12 µm or in a sub-range thereof.

8. Use according to one of claims 1-7,
**characterised in that** the unit for recording the body surface area to be investigated (a) comprises means, e.g. sensors, for detecting and/or monitoring the support position and/or contact pressure of the body area to be investigated, wherein in particular means are provided for detecting and/or monitoring a contact pressure in the range of approximately 0.5-100 N, preferably in the range of approximately 1-50 N and particularly preferably of approximately 20 N are provided.

9. Use according to one of claims 1-8,
**characterised in that** the unit for detecting the body's own IR radiation (c) (ii) comprises at least one first sensor, at least one second sensor and optionally at least one third sensor,
wherein the first sensor is designed to detect IR radiation with a first wavelength or a first wavelength range in the region from about 8 µm to about 12 µm, where the intensity of the body's own IR radiation is substantially independent of the glucose concentration, wherein the second sensor is designed to detect IR radiation with a second wavelength or a second wavelength range in the region from about 8 µm to about 12 µm, where the intensity of the body's own IR radiation changes depending on the glucose concentration, and
wherein the third sensor is designed to reference the body's own IR radiation, wherein the unit for detecting the body's own IR radiation (c) (ii) comprises two or more second sensors that are designed to detect IR radiation at different wavelengths or wavelength ranges, where the intensity of the body's own IR radiation changes depending on the glucose concentration.

10. Use according to one of claims 1-9,
**characterised in that** the unit for detecting radiation (c) is in contact with a thermally conductive carrier, wherein in particular the unit for detecting terahertz radiation (c) (i) and the unit for detecting IR radiation (c) (ii) are in contact with the same thermally conductive carrier.

11. Use according to one of claims 1-10,
**characterised in that** a temperature is provided in the detection unit (c) which is at least 5°C, at least 6°C or at least 7°C and up to 15°C lower than the temperature of the body region to be investigated.

12. Use according to one of claims 1-11,
**characterised in that** the device is provided for a sequential detection of terahertz radiation and IR radiation, wherein in particular the body's own IR radiation is first of all detected without irradiating the body area to be investigated and then the body area to be investigated is irradiated with terahertz radiation and the reflected terahertz radiation is detected.

13. Use according to one of claims 1-12,
**characterised in that** the unit (f) is provided for the temperature-compensated evaluation of the signals on the basis of the temperature values measured and optionally set by the elements (d) and/or (e), and/or
that the unit (f) is provided for the combined evaluation of the signals from the terahertz detection unit (c) (i) and the IR detection unit (c) (ii).

14. Use according to any one of claims 1-13 for the non-invasive quantitative determination of glucose in the blood of a test subject.

15. Method for the non-invasive quantitative determination of glucose in the blood of a test subject, comprising the steps:
(i) irradiating a body region originating from the test subject with terahertz radiation, in particular in a wavelength region from about 0.1 mm to about 5 mm, from about 0.12 mm to about 5 mm or from about 0.1 mm to about 1 mm, and detecting reflected terahertz radiation by a unit for detecting terahertz radiation from the irradiated body region in a wavelength range in which the intensity of the reflected terahertz radiation changes depending on the glucose concentration,
(ii) separately detecting the body's own IR radiation from a body region originating from the test subject by a unit for detecting IR radiation at at least a first wavelength or a first wavelength range in the wavelength region from about 8 µm to about 12 µm, where the intensity of the body's own IR radiation is substantially independent of the glucose concentration, and at least a second wavelength or a second wavelength range in the wavelength region from about 8 µm to about 12 µm, where the intensity of the body's own IR radiation changes depending on the glucose concentration, and optionally non-specific detection of the body's own IR radiation from the irradiated body surface area for referencing,
wherein the temperature in the region of the unit for detecting IR radiation is lower than the temperature of the body area to be investigated,
(iii) combined evaluation of the signals detected according to (i) and (ii) taking account of the temperature of the body area to be investigated and the temperature in the region of the units for detecting terahertz radiation and IR radiation, and wherein optionally a selective evaluation of the body's own IR radiation from capillary blood vessels of the dermis of the body area is performed and
(iv) determining the glucose concentration on the basis of the evaluated signals,
wherein the determination of glucose is preferably carried out by detecting the body's own IR radiation from a fingertip of the test subject without stimulation by an external source of IR radiation.

## Revendications

1. Utilisation d'un dispositif pour la détermination non invasive du glucose dans le sang d'un sujet test, dans lequel le dispositif comprend :
(a) une unité (16) pour le logement d'une partie du corps (14) provenant du sujet test et à examiner,
(b) une source de rayonnement (100) pour la production d'un rayonnement terahertz, plus particulièrement d'un rayonnement terahertz dans un domaine de longueurs d'onde d'environ 0,1 mm à environ 5 mm, d'environ 0,12 mm à environ 5 mm ou d'environ 0,1 mm à environ 1 mm, pour l'irradiation de la partie du corps à examiner,
(c) une unité pour la mesure du rayonnement provenant de la partie du corps à examiner, comprenant :
(i) une unité (118) pour la mesure du rayonnement terahertz réfléchi provenant de la partie du corps à examiner, qui est conçue pour mesurer un rayonnement terahertz dans un domaine de longueurs d'onde dans lequel l'intensité du rayonnement terahertz réfléchi varie en fonction de la concentration du glucose,
(ii) une unité (120) pour la mesure d'un rayonnement IR propre au corps provenant de la partie du corps à examiner, qui est conçue pour la mesure séparée d'un rayonnement IR dans au moins deux longueurs d'onde ou plages de longueurs d'onde différentes d'environ 8 µm à environ 12 µm,
dans lequel, pour une première longueur d'onde ou une première plage de longueurs d'onde, l'intensité du rayonnement IR propre au corps est globalement indépendante de la concentration de glucose et
dans lequel, pour une deuxième longueur d'onde ou une deuxième plage de longueurs d'onde, l'intensité du rayonnement IR propre au corps varie en fonction de la concentration de glucose et
dans lequel l'unité est, le cas échéant, également conçue pour la mesure non spécifique d'un rayonnement IR propre au corps et
(d) -
(i) un élément (16b) pour la mesure de la température dans la partie du corps à examiner,
(ii) le cas échéant, un élément pour le réglage de la température dans la partie du corps à examiner,
(e) -
(i) un élément (26) pour la mesure de la température dans l'unité de mesure (c),
(ii) un élément (28) pour le réglage de la température dans l'unité de mesure (c),
dans lequel il est prévu que la température dans l'unité de mesure (c) est inférieure à la température dans la partie du corps à examiner et
(f) une unité (124) qui est conçue pour l'analyse compensée en température des signaux terahertz provenant de l'unité de mesure (c) (i) et des signaux IR propres au corps provenant de l'unité de mesure (c) (ii) et pour la détermination de la concentration de glucose sur la base des signaux terahertz et des signaux IR propres au corps analysés,
dans lequel l'unité est conçue, le cas échéant, pour l'analyse sélective d'un rayonnement IR propre au corps qui provient des vaisseaux sanguins capillaires du derme de la partie du corps.

2. Utilisation selon la revendication 1,
**caractérisée en ce que** la source de rayonnement comprend, pour la production d'un rayonnement terahertz (b), une antenne, plus particulièrement une antenne de type patch ou dipôle et de préférence
est conçue pour émettre un rayonnement terahertz modulé en fréquence ou modulé par impulsions.

3. Utilisation selon la revendication 1 ou 2,
**caractérisée en ce que**, dans le trajet du rayonnement entre la source de rayonnement pour la production d'un rayonnement terahertz (b) et la partie du corps à examiner, est disposée une lentille de focalisation, par exemple une lentille sphérique, une lentille asphérique ou une lentille de Fresnel, dans laquelle la lentille de focalisation est constituée d'un matériau globalement transparent pour le rayonnement terahertz, dans laquelle la lentille de focalisation est de préférence conçue pour focaliser le rayonnement produit par la source de rayonnement terahertz (b) sur une zone prédéterminée de la partie du corps à examiner, plus particulièrement sur une zone contenant du sang capillaire.

4. Utilisation selon l'une des revendications 1 à 3,
**caractérisée en ce que** l'unité (c) (i) est conçue pour mesurer un spectre à large bande dans le domaine du terahertz, plus particulièrement dans une plage de fréquence d'environ 0,12 mm à environ 5 mm (qui correspond à 60 GHz à environ 2,5 THz) et/ou
l'unité (c) (i) est conçue pour produire une amplification à un ou plusieurs étages, plus particulièrement une amplification à deux étages du rayonnement terahertz réfléchi par la partie du corps à examiner.

5. Utilisation selon l'une des revendications 1 à 4,
**caractérisée en ce que** l'unité (c) (i) est conçue pour la mesure combinée d'un rayonnement terahertz réfléchi provenant de la partie du corps à examiner et d'un rayonnement terahertz introduit dans la partie du corps à examiner et dans laquelle l'unité (c) (i) est conçue plus particulièrement pour la mesure de signaux modulés en fréquence ou modulés par impulsions.

6. Utilisation selon l'une des revendications 1 à 5,
**caractérisée en ce que**
le dispositif ne contient aucune source externe pour la production d'un rayonnement IR.

7. Utilisation selon l'une des revendications 1 à 6,
**caractérisée en ce que** l'unité pour le logement de la partie du corps à examiner (a) comprend un élément au moins partiellement isolant par rapport à l'unité (c) pour la mesure d'un rayonnement, pour l'appui de la partie du corps à examiner, cet élément contenant une zone transparente pour le rayonnement terahertz, plus particulièrement d'environ 0,12 mm à environ 5 mm (correspondant à environ 60 GHz à environ 2,5 THz) ou dans un sous-domaine de celui-ci et pour un rayonnement IR dans le domaine de longueurs d'onde plus particulièrement d'environ 8 µm à 12 µm ou dans un sous-domaine de celui-ci.

8. Utilisation selon l'une des revendications 1 à 7,
**caractérisée en ce que**
l'unité pour le logement de la partie du corps à examiner (a) comprend des moyens, par exemple des capteurs, pour la mesure et/ou le contrôle d'une position d'appui et/ou d'une pression d'appui de la partie du corps à examiner, dans laquelle, plus particulièrement des moyens pour la mesure et/ou le contrôle d'une pression d'appui de l'ordre d'environ 0,5 - 100 N, de préférence de l'ordre d'environ 1 - 50 N et plus particulièrement d'environ 20 N sont prévus.

9. Utilisation selon l'une des revendications 1 à 8,
**caractérisée en ce que**
l'unité pour la mesure d'un rayonnement IR propre au corps (c) (ii) comprend au moins un premier capteur, au moins un deuxième capteur et, le cas échéant, au moins un troisième capteur,
dans laquelle le premier capteur est conçu pour la mesure d'un rayonnement IR avec une première longueur d'onde ou une première plage de longueurs d'onde dans le domaine d'environ 8 µm à environ 12 µm, où l'intensité du rayonnement IR propre au corps est globalement indépendante de la concentration de glucose,
dans laquelle le deuxième capteur est conçu pour la mesure d'un rayonnement IR avec une deuxième longueur d'onde ou une deuxième plage de longueurs d'onde dans le domaine d'environ 8 µm à environ 12 µm, où l'intensité du rayonnement IR propre au corps varie en fonction de la concentration de glucose et
dans laquelle le troisième capteur est conçu pour le référencement du rayonnement IR propre au corps, dans laquelle
l'unité pour la mesure d'un rayonnement IR propre au corps (c) (ii) comprend de préférence deux deuxièmes capteurs ou plus, qui sont conçus pour la mesure d'un rayonnement IR pour différentes longueurs d'onde ou plages de longueurs d'onde, où l'intensité du rayonnement IR propre au corps varie en fonction de la concentration de glucose.

10. Utilisation selon l'une des revendications 1 à 9,
**caractérisée en ce que**
l'unité pour la mesure de rayonnement (c) est en contact avec un support thermiquement conducteur, dans laquelle, plus particulièrement, l'unité pour la mesure d'un rayonnement terahertz (c) (i) et l'unité pour la mesure d'un rayonnement IR (c) (ii) sont en contact avec le même support thermiquement conducteur.

11. Utilisation selon l'une des revendications 1 à 10,
**caractérisée en ce que**
une température dans l'unité de mesure (c) est prévue, qui est inférieure d'au moins 5 °C, d'au moins 6 °C ou d'au moins 7 °C et de jusqu'à 15 °C à la température de la partie du corps à examiner.

12. Utilisation selon l'une des revendications 1 à 11,
**caractérisée en ce que** le dispositif est prévu pour une mesure séquentielle d'un rayonnement terahertz et d'un rayonnement IR, dans laquelle, plus particulièrement, une mesure du rayonnement IR propre au corps est d'abord effectuée sans irradiation de la partie du corps à examiner puis une irradiation de la partie du corps à examiner est effectuée avec un rayonnement terahertz et une mesure du rayonnement terahertz réfléchi est effectuée.

13. Utilisation selon l'une des revendications 1 à 12,
**caractérisée en ce que**
l'unité (f) est prévue pour l'analyse compensée en température des signaux sur la base des valeurs de température mesurées et, le cas échéant, réglées par les éléments (d) et/ou (e) et/ou
l'unité (f) est prévue pour l'analyse combinée des signaux provenant de l'unité de mesure terahertz (c) (i) et de l'unité de mesure IR (c) (ii).

14. Utilisation selon l'une des revendications 1 à 13 pour la détermination non invasive quantitative du glucose dans le sang d'un sujet test.

15. Procédé de détermination non invasive quantitative du glucose dans le sang d'un sujet test, comprenant les étapes suivantes :
(i) irradiation d'une partie du corps provenant du sujet test avec un rayonnement terahertz, plus particulièrement dans un domaine de longueurs d'onde d'environ 0,1 mm à environ 5 mm, d'environ 0,12 mm à environ 5 mm ou d'environ 0,1 mm à environ 1 mm, et mesure du rayonnement terahertz réfléchi par une unité pour la mesure d'un rayonnement terahertz provenant de la partie du corps irradiée dans une plage de longueurs d'onde dans laquelle l'intensité du rayonnement terahertz réfléchi varie en fonction de la concentration de glucose,
(ii) mesure séparée d'un rayonnement IR propre au corps provenant d'une partie du corps du sujet test par une unité pour la mesure d'un rayonnement IR pour au moins une première longueur d'onde ou une première plage de longueurs d'onde dans le domaine de longueurs d'onde d'environ 8 µm à environ 12 µm, où l'intensité du rayonnement IR propre au corps est globalement indépendante de la concentration de glucose, et pour au moins une deuxième longueur d'onde ou une deuxième plage de longueurs d'onde dans le domaine de longueurs d'onde d'environ 8 µm à environ 12 µm, où l'intensité du rayonnement IR propre au corps varie en fonction de la concentration de glucose et, le cas échéant, mesure non spécifique d'un rayonnement IR propre au corps provenant de la partie de la surface du corps irradiée pour un référencement, dans laquelle la température au niveau de l'unité pour la mesure d'un rayonnement IR est inférieure à la température de la partie du corps à examiner,
(iii)analyse combinée des signaux mesurés selon (i) et (ii) en tenant compte de la température de la partie du corps à examiner et de la température au niveau des unités pour la mesure d'un rayonnement terahertz et d'un rayonnement IR et dans laquelle, le cas échéant, une analyse sélective d'un rayonnement IR propre au corps provenant de vaisseaux sanguins capillaires du derme de la partie du corps est effectuée et
(iv)détermination de la concentration de glucose sur la base des signaux analysés,
dans laquelle la détermination du glucose est effectuée de préférence par la mesure d'un rayonnement IR propre au corps provenant du bout d'un doigt du sujet test sans excitation par une source externe de rayonnement IR.
